(19) Europäisches Patentamt
European Patent Office
Office européen des brevets

(11) **EP 1 579 838 A1**

(12) **EUROPEAN PATENT APPLICATION**
published in accordance with Art. 158(3) EPC

(43) Date of publication:
28.09.2005 Bulletin 2005/39

(51) Int Cl.⁷: **A61J 1/05**

(21) Application number: 03778897.3

(22) Date of filing: **12.12.2003**

(86) International application number:
**PCT/JP2003/015974**

(87) International publication number:
**WO 2004/052270 (24.06.2004 Gazette 2004/26)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **12.12.2002 JP 2002360241**
**04.09.2003 JP 2003312541**
**16.09.2003 JP 2003322597**

(71) Applicant: **Asahi Kasei Kabushiki Kaisha**
**Osaka 530-8205 (JP)**

(72) Inventors:
• **HORI, Takahiro**
**Oita-shi, Oita 870-0836 (JP)**

• **SATOU, K.,**
**Asahi-Kasei-Kamiooka-shataku-4-302**
**Yokohama-shi, Kanagawa 233-0007 (JP)**
• **TAKASA, Kenji**
**Yokosuka-shi, Kanagawa 237-0066 (JP)**
• **YANASE, Satoshi**
**Yokohama-shi, Kanagawa 232-0066 (JP)**

(74) Representative: **von Kreisler, Alek et al**
**Deichmannhaus am Dom,**
**Postfach 10 22 41**
**50462 Köln (DE)**

(54) **VIRUS-REMOVING BAG AND VIRUS-REMOVING METHOD USING THE SAME**

(57) A virus removal bag for removing viruses from a virus-containing suspension, comprising a pouchy casing (a) having at least one inlet and at least one outlet, and a separation membrane (b) which is securely held in the pouchy casing (a) and which partitions the internal space of the pouchy casing (a) into a first compartment (c) communicating with the inlet and a second compartment (d) communicating with the outlet, wherein at least a part of the separation membrane (b) is made of a virus removal membrane.

## Fig. 1

EP 1 579 838 A1

**Description**

BACKGROUND OF THE INVENTION

Field of the Invention

The present invention relates to a virus removal bag. More particularly, the present invention is concerned with a virus removal bag comprising a pouchy casing (a) having at least one inlet for a virus -containing suspension and at least one outlet for a virus-removed suspension; and a separation membrane (b) which is securely held in the pouchy casing (a) and which partitions the internal space of the pouchy casing (a) into a first compartment (c) communicating with the inlet and a second compartment (d) communicating with the outlet, wherein at least a part of the separation membrane (b) is made of a virus removal membrane for removing viruses from a virus-containing suspension by filtration to obtain a filtrate which is a virus -removed suspension, and wherein the first compartment (c) serves to receive a virus-containing suspension introduced through the inlet, and the second compartment (d) serves to collect the filtrate obtained by filtering the virus-containing suspension through the virus removal membrane. The present invention is also concerned with a method for removing viruses by using the above-mentioned virus removal bag. When the virus removal bag of the present invention is used for removing viruses from a virus-containing suspension, a filtrate which is a virus-removed suspension is collected inside the virus removal bag and, therefore, there is no need to provide a separate container for receiving the filtrate. Accordingly, the virus removal bag of the present invention is advantageous not only in that it has a simple structure, but also in that the virus removal can be achieved by easy operations. The present invention is also concerned with a method for preparing a virus-removed plasma. The method of the present invention is advantageous in that all viruses, including viruses in the window period and viruses which are not covered by the virus detection methods employed in the field of blood treatment, can be easily removed from plasma without any of performing complicated aseptic operations and using large scale apparatuses. Therefore, by the use of the method of the present invention, a transfusable plasma having very low risk of viral infection can be easily prepared at low cost.

Prior art

Human or animal plasma is useful not only for transfusion, but also as a raw material for plasma products and plasma-derived products and as a material used in various biotechnological applications. However, plasma carries a potential risk of viral contamination. Especially when a transfusable plasma prepared from donated blood is contaminated with viruses, a patient receiving such a virus-contaminated plasma faces a high risk of viral infection.

As a means for preventing viral infection from donated blood, there can firstly be mentioned a screening analysis. This screening analysis is comprised of a donor questionnaire and an infection analysis of donated blood. With respect especially to viruses which cause severe infections, an analysis based on an antigen-antibody reaction is performed. With respect to several viruses including hepatitis B virus, hepatitis C virus and AIDS virus, a manual analysis by a person is performed in addition to a donated blood analysis by an automatic blood analyzer, and any blood showing a positive reaction is discarded.

The possibility of viral infection from donated blood has not been satisfactorily eliminated by the above-mentioned methods used today. In the process for preparing a plasma-derived products, plasma component is subjected to several purification steps before final treatment with a filtration membrane having a virus removal ability. Therefore, the thus obtained plasma -derived products have only minimal risk of viral infection. On the other hand, during the process for producing a transfusable plasma, some viruses contained in blood are possibly undetectable by infection analyses and, therefore, it is very difficult to completely eliminate the infection risk of blood transfusion. Viruses contained in blood are undetectable by an infection analysis when the viruses are those which are not covered by virus detection methods employed and when the viruses are in the so-called "window period". During the window period, a virus is insusceptible to an antigen-antibody reaction which is specific therefor. When the viruses contained in donated blood are in the window period, such blood will pass the screening analysis.

As another means for eliminating the possibility of viral infection from donated blood, there have been developed techniques for inactivating the viruses contained in donated blood. Among the conventional techniques for inactivating the viruses, two well known methods are the solvent/detergent treatment in which collected blood is brought into contact with a solution containing a surfactant, and the photochemical inactivation treatment in which the viruses contained in collected blood are caused to take in a reactive substance (such as a psoralen derivative) and the viruses containing the reactive substance are subjected to active light irradiation to thereby destroy the genome of the viruses by a photochemical reaction. However, the above-mentioned methods are not effective for all types of viruses. Further, there are problems, such as low safety of the substances used for inactivating the viruses and the increase in the cost for preparing transfusable blood or blood components.

The viruses contained in blood can be removed by filtration using a filter having a pore diameter such as can prevent the viruses from passing through the filter. Filtration-removal can be effectively performed irrespective of the type of viruses contained in blood (i.e., whether or not the virus is covered by the virus detection method employed) and irrespective of the infection stage of viruses (i.e., whether or not the virus is in the window period). For example, Unexamined Japanese Patent Application Laid-Open Specification Nos. Sho 62-67456 and Sho 63-88130 each disclose a system for removing viruses from plasma by using a filter made of hollow fibers. Specifically, Unexamined Japanese Patent Application Laid-Open Specification No. Sho 62-67456 describes a method in which blood is introduced into a filter unit made of hollow fibers and the blood is filtered therethrough by using a centrifugal force for driving the filtration. Further, Unexamined Japanese Patent Application Laid-Open Specification No. Sho 63-88130 describes a method in which donated blood (whole blood) is introduced into a blood bag and a plasma component is separated from the whole blood by centrifugation, followed by passing the separated plasma component through a virus removal filter.

However, when a virus removal step using a filter unit as mentioned above is incorporated into a conventional blood treatment method comprising a step of subjecting blood collected in a blood bag to centrifugation to separate the blood into a blood cell component and a plasma component, the resultant method has the following problems. With respect to the above -mentioned filter unit which is packed with hollow fibers, the casing of the filter unit is made of a hard material, thus posing a problem in that, when the blood bag containing whole blood is subjected to centrifugation for separating whole blood into blood components under conditions wherein the blood bag is connected to the filter unit, there is a danger that the blood bag is damaged by the casing of the filter unit. For solving this problem, it becomes necessary to perform an operation in which only the blood bag containing whole blood (and not having the filter unit connected thereto) is centrifuged and, then, the filter unit is aseptically connected to the centrifuged blood bag. However, in the actual site where the blood treatment is performed, it is usually difficult to provide an aseptic area for aseptically connecting the filter unit to the blood bag. In addition, when the aseptic conditions of the aseptic area are broken, for example, by the moving of people into and out of the aseptic area, performance of a step for connecting a filter unit to a blood bag under such non-aseptic conditions may become another cause of infection.

Further, connecting a filter unit to a blood bag as in the above-mentioned method means that one filter unit is used per donor to obtain donated blood and, thus, every filter unit is disposed of upon a single use thereof for treating only 200 to 400 ml (which is the amount of blood obtained per donation) of donated blood. It is preferred that a disposable filter unit has a structure which is as simple as possible so as to lower the production cost thereof. From such a viewpoint, a filter unit using hollow fibers is not always suitable as a disposable filter unit. As an example of a filter unit having a more simple structure, there can be mentioned a leukocyte removal filter unit disclosed in Unexamined Japanese Patent Application Laid-Open Specification No. Hei 7-267871. This filter unit has a structure wherein a filtration medium made of a nonwoven fabric for the adsorption removal of leukocytes is accommodated in a flexible housing. However, when this filter unit disclosed in Unexamined Japanese Patent Application Laid-Open Specification No. Hei 7-267871 is used for filtration by centrifugal force, it is necessary that a blood bag containing blood before filtration, the filter unit and a blood collection bag for collecting the filtered blood be serially arranged and connected in this order through tubes in a direction in which the centrifugal force is exerted. A very large space is necessary for arranging the bags and the filter unit in such a manner and, in addition, there is a problem that the positional relationships between the bags and the filter unit become disturbed during the centrifugation, thus rendering difficult a stable centrifugal operation. Therefore, even when the filtration medium used in the filter unit disclosed in Unexamined Japanese Patent Application Laid-Open Specification No. Hei 7-267871 is replaced by a filtration medium suitable for removing viruses, the above-mentioned problems of the leukocyte removal unit remain unsolved, that is, a large space is necessary for centrifugal filtration and the stable centrifugal operation becomes difficult due to the disturbance of the positional relationships between the bags and the filter unit during the centrifugation.

Further, when plasma obtained from donated blood is subjected to filtration, especially when there is used a filter having a very small pore diameter for cutting off viruses, clogging of the filter by lipids and the like contained in plasma becomes a serious problem. Such a problem does not occur in the production of a plasma-derived product, in which the plasma is subjected to several purification steps and the filtration is performed with respect to purified plasma containing almost no substances which are causative of clogging. Plasma obtained immediately after centrifugation of donated blood for separating plasma from blood cell components is called "fresh plasma", and wide individual variation exists in the composition of the fresh plasma. Especially, plasma of a person suffering from or having a high risk of hyperlipemia may contain lipid components in an amount such that visually observable large globules of lipids are formed in the plasma when the plasma is cooled to room temperature. When such plasma is treated using a membrane, a lowering of the amount of treated plasma due to the clogging of the membrane frequently becomes a problem. As a method for solving this problem, Unexamined Japanese Patent Application Laid-Open Specification No. Hei 3-146067 discloses a method in which multiple filter units having different pore diameters are connected together. However, when a system for preparing a transfusable plasma from donated blood (namely, a system for separating plasma from donated blood and removing viruses from the separated plasma) is produced using this method, it becomes necessary to connect together multiple filter units each employing hollow fibers. As a result, a large part of the length of the virus removal system is constituted by the filter units, thus rendering difficult the centrifugation operation. Further, there is a practical problem in that the costs for the filter units become high.

As apparent from the above, there is no simple system or method for virus removal, which is compatible with the conventional blood treatment systems and which enables a treatment in which fresh plasma obtained immediately after blood donation is treated for removing all viruses including viruses in the window period and viruses which are not covered by the virus detection methods employed in the field of blood treatment.


SUMMARY OF THE INVENTION

In this situation, the present inventors have conducted extensive and intensive studies for solving the above-mentioned problems. As a result, it has unexpectedly been found that, when viruses are removed from a virus-containing suspension by using a virus removal bag comprising a pouchy casing (a) having an inlet and an outlet, and a separation membrane (b) which is securely held in the pouchy casing (a) and which partitions the internal space of the pouchy casing (a) into a first compartment (c) communicating with the inlet and a second compartment (d) communicating with the outlet, wherein at least a part of the separation membrane (b) is made of a virus removal membrane, the virus-containing suspension is collected in the first compartment (c), followed by filtration through the virus removal membrane which constitutes at least a part of the separation membrane (b), and a filtrate which is a virus-removed suspension is collected in the second compartment (d). When viruses are removed from a virus-containing suspension by using a virus removal bag having the above-mentioned structure, a filtrate which is a virus-removed suspension is collected inside the virus removal bag. By virtue of this property, the virus removal bag is advantageous not only in that it has a simple structure, but also in that the virus removal can be achieved by easy operations. Further, the present inventors have found that, by using a virus removal system obtained by incorporating the above -mentioned virus removal bag into a conventional plasma treatment system, a transfusable plasma having very low risk of viral infection can be easily prepared at low cost without the need for complicated aseptic operations or for large scale apparatuses, the prepared transfusable plasma being free of all viruses including viruses in the window period and viruses which are not covered by the virus detection methods employed in the field of blood treatment. The present invention has been completed based on these novel findings.

Accordingly, it is an object of the present invention to provide a virus removal bag which enables virus removal by easy operations without using a separate container for receiving a filtrate.

It is another object of the present invention to provide a method for removing viruses from a virus -containing suspension by using the above-mentioned virus removal bag.

It is still another object of the present invention to provide a method for obtaining a virus-removed plasma by using the above-mentioned virus removal bag.

The foregoing and other objects, features and advantages of the present invention will be apparent to those skilled in the art from the following detailed description taken in connection with the accompanying drawings and appended claims.


BRIEF DESCRIPTION OF THE DRAWINGS

In the drawings:

Fig. 1 shows a schematic plan view of an example of the virus removal bag of the present invention having a separation membrane (b) in the form of a sheet;
Fig. 2 shows schematic views explaining an example of the virus removal bag of the present invention having a separation membrane (b) in the form of a membranous, overall surrounding wall of a filter bag, wherein Fig. 2(a) is a plan view of the virus removal bag, and Fig. 2(b) is a cross-sectional view taken along line II-II of Fig. 2(a);
Fig. 3 shows a schematic cross-sectional view of an example of a composite filter used as a virus removal membrane;
Fig. 4 shows a schematic cross-sectional view of an example of a composite filter used as a virus removal membrane;
Fig. 5 shows a schematic plan view of an example of a composite filter used as a virus removal membrane, in which an adhesion area provided for unifying the laminated filter materials and a nonwoven fabric is shown;
Fig. 6 shows schematic views explaining an example of a filter bag used as a separation membrane (b), wherein each of Fig. 6(a) to Fig. 6(c) shows a filter bag in which all of its overall surrounding wall is made of a virus removal membrane, and Fig. 6(d) shows a filter bag in which a part of its overall surrounding wall is made of a virus removal membrane and the remaining part of its overall surrounding wall is made of a sheet which is impervious to a virus-containing suspension;

Fig. 7 shows schematic views explaining the portion of the virus removal bag of the present invention which is used to determine the volume of the second compartment (d), wherein Fig. 7(a) is a plan view of a virus removal bag containing separation membrane (b) in the form of a sheet, Fig. 7(b) is a plan view of a virus removal bag having separation membrane (b) in the form of a membranous, overall surrounding wall of a filter bag, and Fig. 7 (c) is a cross-sectional view taken along line VII-VII of Fig. 7(b);

Fig. 8 shows schematic views explaining the joint between a separation membrane (b) in the form of a sheet and a pouchy casing (a) in the virus removal bag of the present invention, wherein Fig. 8(a) shows a joint formed between a separation membrane (b) in the form of a sheet and an inner wall of a pouchy casing (a), and Fig. 8(b) shows a joint formed by adhering peripheral portions of at least two sheets forming a pouchy casing (a) to each other through a peripheral portion of a separation membrane (b) in the form of a sheet;

Fig. 9 shows schematic views explaining the method for connecting a filter bag and a pouchy casing (a) for obtaining the virus removal bag of the present invention, wherein Fig. 9(a) is a virus removal bag in which a filter bag and a pouchy casing (a) are connected to each other only through tube 13 used as an inlet, and Fig. 9(b) is a virus removal bag in which a filter bag and a pouchy casing (a) are connected to each other along the entire upper peripheral portion of the pouchy casing (a);

Figs. 10(a) to 10(f) each show a plan view of an example of a filter bag which is tapered toward the forward end of the filter bag as viewed in a flow direction of a virus-containing suspension in the virus removal bag;

Fig. 11 shows schematic views explaining an example of a virus removal bag containing a spongy adsorber in a first compartment (c), wherein Fig. 11(a) is a plan view of the virus removal bag, and Fig. 11(b) is a cross-sectional view taken along line XI-XI of Fig. 11(a);

Fig. 12 shows schematic views explaining an example of a spacer used in the present invention, wherein Fig. 12 (a) is a plan view of a spacer containing a filter bag therein, and Fig. 12(b) is a cross-sectional view taken along line XII-XII of Fig. 12(a);

Figs. 13 to 22 each show a schematic view explaining an example of a closed, virus removal system which can be used in the method of the present invention for obtaining a virus-removed plasma;

Fig. 23 shows a schematic view explaining an example of a virus removal bag placed in a centrifuge cup;

Fig. 24 shows schematic views explaining an example of a securing hook used for securing a virus removal bag to a centrifuge cup, wherein Fig. 24(a) is a schematic plan view of a securing hook, and Fig. 24(b) is a schematic side view of a securing hook;

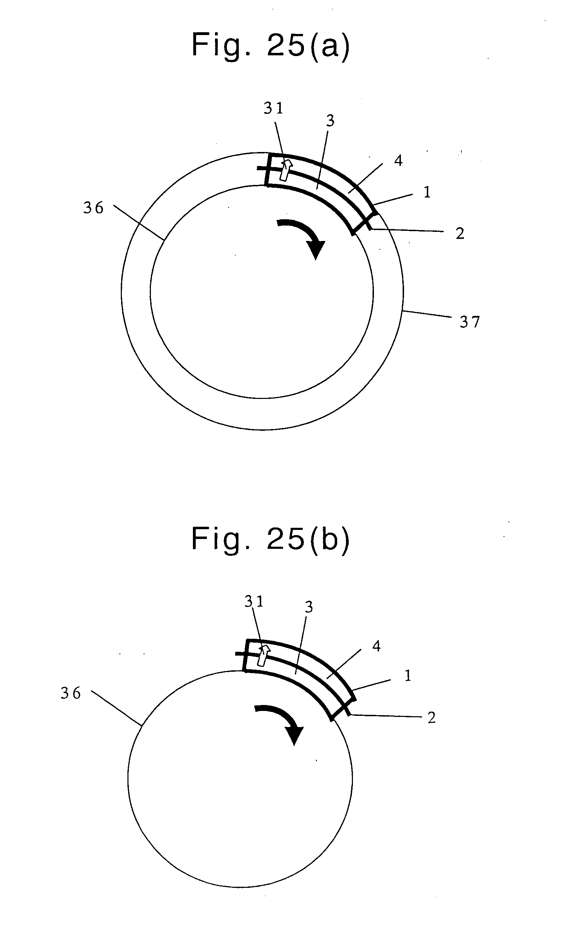

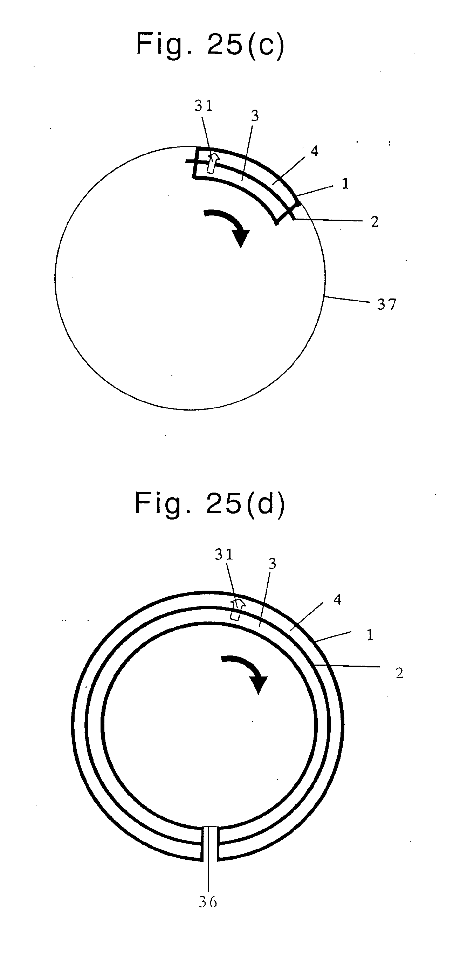

Fig. 25 shows schematic views explaining examples of methods for promoting the filtration by applying a centrifugal force to a virus removal bag, wherein Fig. 25(a) is a schematic view of two rotating elements having a virus removal bag attached therebetween, Fig. 25(b) is a schematic view of one rotating element having a virus removal bag attached to an outer side surface thereof, Fig. 25(c) is a schematic view of one rotating element having a virus removal bag attached to an inner side surface thereof, and Fig. 25(d) is a schematic view of one rotating element having a virus removal bag attached to an outer side surface thereof, wherein the length of the virus removal bag is almost the same as the circumference of the rotating element;

Fig. 26 shows a schematic view explaining an example of a virus removal bag placed in a centrifuge cup;

Fig. 27 shows a schematic view explaining an example of a method for promoting the filtration by using a roll-type pressurizing apparatus for applying a pressure to a virus removal bag;

Fig. 28 shows a schematic view explaining an example of a method for promoting the filtration by using a plate-type pressurizing apparatus for applying a pressure to a virus removal bag;

Fig. 29 shows a schematic view explaining an example of a method for pressurizing a virus removal bag by using a bag containing a gas for pressurization;

Fig. 30 shows a schematic view explaining the structure of the virus removal bag produced in Example 7;

Fig. 31 shows a schematic view explaining the structure of a cylindrical, hard polysulfone casing used in Reference Example 2, wherein the casing is used as a dummy of a conventional hollow fiber filter module; and

Fig. 32 shows a schematic view explaining the shape of the filter bag produced in Example 8.

Description of Reference Numerals

1          pouchy casing (a)
2          separation membrane (b) in the form of a sheet
3          first compartment (c)
4          second compartment (d)
5          inlet
6          outlet

| 7 | filter bag |
|---|---|
| 7a | virus removal membrane |
| 7b | sheet which is impervious to virus-containing suspension |
| 8 | virus removal filter |
| 9 | prefilter |
| 10 | nonwoven fabric |
| 11 | composite filter |
| 12 | adhesion area |
| 13 | tube used as an inlet |
| 14 | sealed portion of pouchy casing (a) |
| 15 | joint between separation membrane (b) and pouchy casing (a) |
| 16 | spongy adsorber |
| 17 | spacer |
| 18 | cannula |
| 19 | portion of liquid transfer pipe to be melt -sealed and cut |
| 20 | blood bag for whole blood |
| 21 | blood bag for filtered plasma |
| 22 | leukocyte removal unit |
| 23 | blood bag for leukocyte-removed blood |
| 24 | blood bag for buffy coat |
| 25 | bag containing additives and the like |
| 26 | bag for removing air from virus removal bag |
| 27 | bag containing gas for pressurization |
| 28 | centrifuge cup |
| 29 | securing hook |
| 30 | securing bar |
| 31 | direction of centrifugal force |
| 32 | hook |
| 33 | holding plate |
| 34 | securing screw |
| 35 | upper portion of virus removal bag |
| 36, 37 | rotating element |
| 38 | auxiliary pot |
| 39 | roll of a roll-type pressurization apparatus |
| 40 | pinchcock |
| 41 | plate of a plate-type pressurization apparatus |
| 42 | fixation collar |
| 43 | cylindrical, hard polysulfone casing |

DETAILED DESCRIPTION OF THE INVENTION

In one aspect of the present invention, there is provided a virus removal bag for removing viruses from a virus-containing suspension, comprising:

a pouchy casing (a) having at least one inlet for a virus-containing suspension and at least one outlet for a virus-removed suspension; and
a separation membrane (b) which is securely held in the pouchy casing (a) and which partitions the internal space of the pouchy casing (a) into a first compartment (c) communicating with the inlet and a second compartment (d) communicating with the outlet,

wherein at least a part of the separation membrane (b) is made of a virus removal membrane for removing viruses from a virus-containing suspension by filtration to obtain a filtrate which is a virus-removed suspension, and
wherein the first compartment (c) serves to receive a virus-containing suspension introduced through the inlet, and the second compartment (d) serves to collect the filtrate obtained by filtering the virus -containing suspension through the virus removal membrane.

For an easy understanding of the present invention, the essential features and various preferred embodiments of the present invention are enumerated below.

1. A virus removal bag for removing viruses from a virus-containing suspension, comprising:

a pouchy casing (a) having at least one inlet for a virus-containing suspension and at least one outlet for a virus-removed suspension; and
a separation membrane (b) which is securely held in the pouchy casing (a) and which partitions the internal space of the pouchy casing (a) into a first compartment (c) communicating with the inlet and a second compartment (d) communicating with the outlet,

wherein at least a part of the separation membrane (b) is made of a virus removal membrane for removing viruses from a virus-containing suspension by filtration to obtain a filtrate which is a virus-removed suspension, and
wherein the first compartment (c) serves to receive a virus-containing suspension introduced through the inlet, and the second compartment (d) serves to collect the filtrate obtained by filtering the virus -containing suspension through the virus removal membrane.

2. The virus removal bag according to item 1 above, wherein the separation membrane (b) is in the form of a membranous, overall surrounding wall of a filter bag,
wherein the filter bag is securely held in the pouchy casing (a), such that the membranous, overall surrounding wall of the filter bag partitions the internal space of the pouchy casing (a) into the first compartment (c) which is the internal space of the filter bag communicating with the inlet and the second compartment (d) which is the internal space of the pouchy casing (a), exclusive of a filter bag portion, communicating with the outlet, and
wherein at least a part of the surrounding wall of the filter bag is made of a virus removal membrane for removing viruses from the virus-containing suspension by filtration.

3. The virus removal bag according to item 2 above, wherein the filter bag is tapered toward the forward end of the filter bag as viewed in a flow direction of the virus-containing suspension in the virus removal bag, wherein the tapering begins at the backward end of the filter bag or at a portion during the course toward the forward end of the filter bag.

4. The virus removal bag according to any one of items 1 to 3 above, wherein the virus removal membrane is a composite filter in which at least one prefilter and at least one virus removal filter are laminated in this order as viewed in a flow direction of the virus -containing suspension, to thereby form a composite filter, wherein, on at least one side of the composite filter, a nonwoven fabric is provided.

5. The virus removal bag according to any one of items 1 to 4 above, wherein the virus removal membrane is a porous membrane having an average pore diameter in the range of from 1 nm to 100 nm.

6. The virus removal bag according to any one of items 1 to 5 above, wherein the virus removal membrane is a hydrophilic porous membrane obtained by an addition of a hydrophilic compound onto the surface of a porous membrane.

7. The virus removal bag according to item 6 above, wherein the addition of a hydrophilic compound is a graft polymerization reaction of a hydrophilic monomer.

8. The virus removal bag according to any one of items 1 to 7 above, which is flexible.

9. The virus removal bag according to any one of items 1 to 8 above, wherein the second compartment (d) has a volume sufficient to collect all of the filtrate obtained by filtering the virus-containing suspension through the virus removal membrane.

10. The virus removal bag according to any one of items 1 to 9 above, wherein the first compartment (c) contains a spongy adsorber.

11. The virus removal bag according to any one of items 1 to 10 above, wherein the second compartment (d) has a volume in the range of from 100 to 800 cm$^3$.

12. The virus removal bag according to any one of items 1 to 11 above, which is aseptically and fluid -tightly connected to at least one functional bag which has a function other than a virus removal function, thereby providing a closed, multi-bag virus removal system.

13. A method for removing viruses from a virus -containing suspension, comprising:

> (1) providing at least one virus removal bag of any one of items 1 to 12 above;
> (2) introducing a virus-containing suspension to the virus removal bag through the inlet, to receive the virus-containing suspension in the first compartment (c);
> (3) filtering the virus-containing suspension through the virus removal membrane, to thereby remove viruses from the suspension;
> (4) collecting a filtrate which is a virus -removed suspension in the second compartment (d); and
> (5) withdrawing the virus-removed suspension through the outlet.

14. The method according to item 13 above, wherein, in step (3), the filtration of the virus-containing suspension through the virus removal membrane is promoted by applying a centrifugal force to the virus-containing suspension in the first compartment (c).

15. The method according to item 13 above, wherein, in step (3), the filtration of the virus-containing suspension through the virus removal membrane is promoted by applying a pressure to the virus-containing suspension in the first compartment (c).

16. The method according to any one of items 13 to 15 above, wherein the virus-containing suspension is whole blood.

17. The method according to any one of items 13 to 15 above, wherein the virus-containing suspension is plasma.

18. The method according to item 17 above, wherein the plasma has never been frozen.

19. The method according to item 17 or 18 above, wherein the plasma is leukocyte-removed plasma.

20. The method according to any one of items 13 to 19 above, wherein, in step (4), all of the filtrate obtained by filtering the virus-containing suspension through the virus removal membrane is collected in the second compartment (d) before performing step (5).

21. A method for preparing a virus-removed plasma, comprising:

> (1) providing a closed, virus removal system which comprises:

>> a plasma collector means (i) for collecting whole blood comprising plasma and blood cells and separating and collecting plasma from whole blood, wherein the whole blood is suspected to contain viruses,
>> at least one virus removal bag (ii) of any one of items 1 to 11 above, and
>> a plasma recovery means (iii) for recovering a virus-removed plasma,
>> the plasma collector means (i) being aseptically and fluid-tightly connected to the virus removal bag (ii), and the virus removal bag (ii) being aseptically and fluid-tightly connected to the plasma recovery means (iii);

> (2) collecting whole blood from a donor into the plasma collector means (i);
> (3) separating the collected whole blood into plasma and blood cells by centrifugation;
> (4) introducing the separated plasma into the at least one virus removal bag (ii) from the plasma collector means (i), to receive the separated plasma in the first compartment (c) of the virus removal bag (ii);
> (5) filtering the separated plasma through the virus removal membrane of the virus removal bag (ii), to thereby remove viruses from the separated plasma;
> (6) collecting a filtrate which is a virus -removed plasma in the second compartment (d) of the virus removal bag (ii); and
> (7) discharging the virus-removed plasma from the virus removal bag (ii) into the plasma recovery means (iii).

22. The method according to item 21 above, wherein, in step (6), all of the filtrate obtained by filtering the separated plasma is collected in the second compartment (d) of the virus removal bag (ii) before performing step (7).

23. Human or animal plasma obtained by the method of item 21 or 22 above.

Hereinbelow, the present invention will be described in more detail.

The present invention provides a virus removal bag for removing viruses from a virus-containing suspension, comprising:

a pouchy casing (a) having at least one inlet for a virus-containing suspension and at least one outlet for a virus-removed suspension; and
a separation membrane (b) which is securely held in the pouchy casing (a) and which partitions the internal space of the pouchy casing (a) into a first compartment (c) communicating with the inlet and a second compartment (d) communicating with the outlet,

wherein at least a part of the separation membrane (b) is made of a virus removal membrane for removing viruses from a virus-containing suspension by filtration to obtain a filtrate which is a virus-removed suspension, and
wherein the first compartment (c) serves to receive a virus-containing suspension introduced through the inlet, and the second compartment (d) serves to collect the filtrate obtained by filtering the virus -containing suspension through the virus removal membrane.

The virus removal bag of the present invention comprises a pouchy casing (a) having at least one inlet for a virus-containing suspension and at least one outlet for a virus-removed suspension; and a separation membrane (b) which is securely held in the pouchy casing (a) and which partitions the internal space of the pouchy casing (a) into a first compartment (c) communicating with the inlet and a second compartment (d) communicating with the outlet. There is no particular limitation with respect to the pouchy casing (a) used in the virus removal bag of the present invention as long as the casing has at least one inlet and at least one outlet and has a structure such that a solution can be maintained therein. Further, there is no particular limitation with respect to the material used for producing the pouchy casing (a) as long as the material is impervious to a suspension, such as a virus-containing suspension (or a virus-removed suspension), and it is preferred that the pouchy casing (a) is made of a' flexible material mentioned below. With respect to the size of the pouchy casing (a), there is no particular limitation as long as the pouchy casing (a) is capable of securely holding a separation membrane (b) in the internal space thereof, and the size of the pouchy casing (a) may be determined based on the size of the separation membrane (b) and the volume of the virus-containing suspension to be treated.

The separation membrane (b) of the virus removal bag of the present invention may be in the form of a sheet (i.e., a flat membrane) or, alternatively, in the form of a membranous, overall surrounding wall of a filter bag. (Hereinafter, "the separation membrane (b) in the form of a membranous, overall surrounding wall of a filter bag" is frequently referred to simply as "separation membrane (b) in the form of a bag" or "filter bag".) The virus removal bag containing a separation membrane (b) in the form of a bag is a virus removal bag containing a separation membrane (b) in the form of a membranous, overall surrounding wall of a filter bag, wherein the filter bag is securely held in the pouchy casing (a), such that the membranous, overall surrounding wall of the filter bag partitions the internal space of the pouchy casing (a) into the first compartment (c) which is the internal space of the filter bag communicating with the inlet and the second compartment (d) which is the internal space of the pouchy casing (a), exclusive of a filter bag portion, communicating with the outlet, and wherein at least a part of the surrounding wall of the filter bag is made of a virus removal membrane for removing viruses from the virus-containing suspension by filtration. In the present invention, the "membranous, overall surrounding wall of the filter bag" is a membrane which constitutes the filter bag and separates the internal space of the filter bag from the outside.

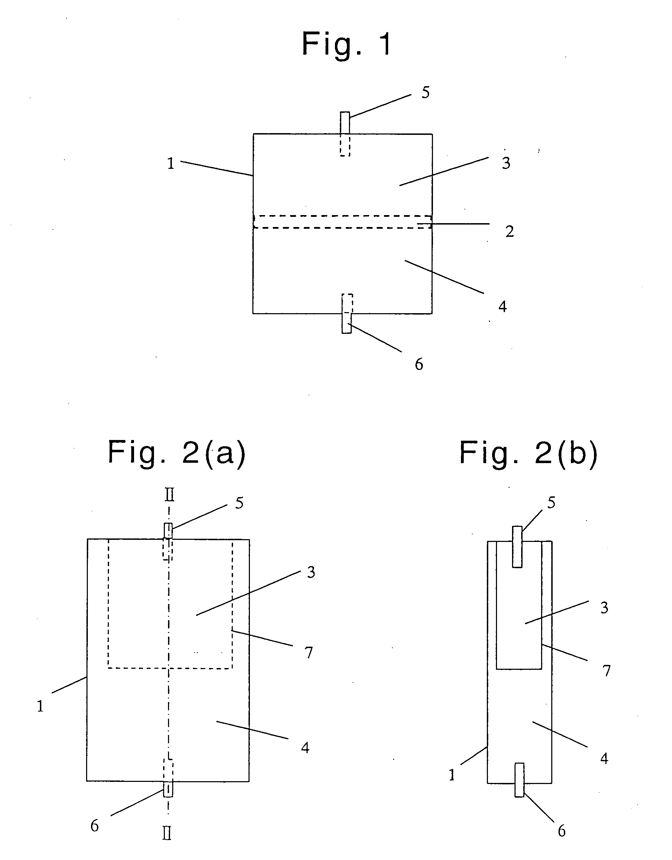

The virus removal bags of the present invention are shown in Figs. 1 and 2. Fig. 1 shows an example of a virus removal bag having a separation membrane (b) in the form of a sheet, and the virus removal bag comprises pouchy casing 1, separation membrane 2, first compartment 3, second compartment 4, and inlet 5 and outlet 6 which are tubes. Fig. 2 shows an example of a virus removal bag having a separation membrane (b) in the form of a bag (filter bag), wherein Fig. 2(a) is a plan view of the virus removal bag, and Fig. 2(b) is a cross-sectional view taken along line II-II of Fig. 2(a). The virus removal bag shown in Fig. 2 comprises pouchy casing 1, filter bag 7, first compartment 3, second compartment 4, and inlet 5 and outlet 6 which are tubes.

At least a part of the separation membrane (b) of the virus removal bag of the present invention is made of a virus removal membrane for removing viruses from a virus-containing suspension by filtration. The expression "at least a part of a separation membrane (b) is made of a virus removal membrane" means that at least 10 %, preferably at least 20 % of the area (of the separation membrane (b)) which contacts the virus -containing suspension is made of a virus removal membrane. Therefore, a virus removal membrane may constitute the whole of the separation membrane (b), but alternatively, the separation membrane (b) may have a structure wherein only a part of the separation membrane (b) is made of a virus removal membrane and the remaining part of the separation membrane (b) is made of a sheet which is impervious to a virus-containing suspension. A sheet which is impervious to a virus -containing suspension is explained below in connection with the filter bag.

The virus removal membrane constituting at least a part of the separation membrane (b) is a membrane which is capable of preventing viruses contained in the virus -containing suspension from permeating through the membrane, and many virus removal membranes having such ability are known in the art. There is no particular limitation with respect to the virus removal membrane used in the present invention, and any conventional virus removal filter can be used. A virus removal filter as such can be used as the virus removal membrane, but it is preferred that the virus removal membrane is a composite filter in which at least one prefilter and at least one virus removal filter are laminated in this order as viewed in a flow direction of the virus -containing suspension, to thereby form a composite filter, wherein, on at least one side of the composite filter, a nonwoven fabric is provided. By the use of such a composite filter, it becomes possible to obtain a virus removal bag which has satisfactory mechanical strength and which can be used to perform filtration stably for a long period of time.

In the present invention, it is preferred that the average pore diameter of the virus removal membrane is in the range of from 1 to 100 nm, more advantageously from 10 to 80 nm, most advantageously 30 to 70 nm. When the average pore diameter of the membrane is in the range of from 1 to 100 nm, viruses can be removed from a virus-containing suspension. The viruses, such as AIDS virus (HIV, average particle diameter: 100 to 120 nm), may be present in plasma and cause serious symptoms to the human body. In the present invention, the average pore diameter of a membrane is a pore diameter as measured in accordance with the methods prescribed under ASTM F316-86 and E128-61. For precisely evaluating the ability of a virus removal membrane to remove viruses, there can be used the log reduction value. The log reduction value (LRV) as the performance of a virus removal membrane is represented by the following formula: $-\log_{10}$ (virus concentration of a filtrate which is a virus-removed suspension)/(virus concentration of a virus-containing suspension before filtration). In the present invention, it is preferred that the LRV is in the range of from 3 to 10, more advantageously from 4 to 9. The log reduction value as the performance of a virus removal membrane is determined as follows. MDBK cells cultured in Dulbecco's MEM containing 5 % equine serum are infected with bovine viral diarrhea virus, to thereby form a culture supernatant of virus-infected cells, and the culture supernatant is collected to thereby obtain a virus -containing suspension. A part of the obtained virus -containing suspension is filtered through a virus removal membrane under conditions wherein the pressure is 0.1 MPa and the temperature is 25 °C, and the resultant filtrate is collected in the form of 10 fractions each containing 2 ml of the filtrate. 1 ml of a sample is obtained from each of the 10 fractions and mixed together, thereby obtaining a virus-removed suspension. The virus concentration of each of the virus-containing suspension (before filtration) and the virus-removed suspension (which is a filtrate) is determined by the $TCID_{50}$ method using cultured MDBK cells, and the log reduction value is calculated based on the determined virus concentrations of the virus-containing suspension and the virus-removed suspension.

The composite filter used in the present invention has at least one prefilter and at least one virus removal filter which are laminated in this order as viewed in a flow direction of the virus-containing suspension. It is preferred that the prefilter used in the present invention has an average pore diameter which is larger than that of the virus removal membrane. Specifically, it is preferred that the average pore diameter of the prefilter is 1.2 to 10.0 times the average pore diameter of the virus removal filter. Further, the thickness of both the virus removal filter and the prefilter is preferably in the range of from 5 to 500 μm, more preferably from 10 to 200 μm. With respect to the virus removal filter and the prefilter, when the average pore diameter and the thickness are respectively in the above-mentioned ranges, both the virus removal filter and the prefilter exhibit sufficient strength for practical use, and use of such virus removal filter and such prefilter prevents a lowering of the flow rate of liquid filtration from occurring with time during the filtration.

The composite filter used in the present invention is provided with a nonwoven fabric on at least one side thereof. The nonwoven fabric is provided for protecting the virus removal filter and the prefilter and for capturing large particles present in the virus -containing suspension (e.g., when the virus-containing suspension is plasma, the large particles are lipids and the like). The nonwoven fabric used in the composite filter is preferably made of a resin selected from the group consisting of a polyvinyl chloride, a polypropylene, a polyethylene, a polyethylene terephthalate, a nylon, a polyurethane, a styrene-isobutylene-styrene copolymer, a cellulose, a cellulose acetate and a mixture thereof. For obtaining sufficient strength to protect the filters and for effectively capturing the particles and lipids, the weight per unit area of the nonwoven fabric is preferably in the range of from 1 to 100 $g/m^2$, and the diameter of the fibers of the nonwoven fabric is preferably in the range of from 0.3 to 100 μm.

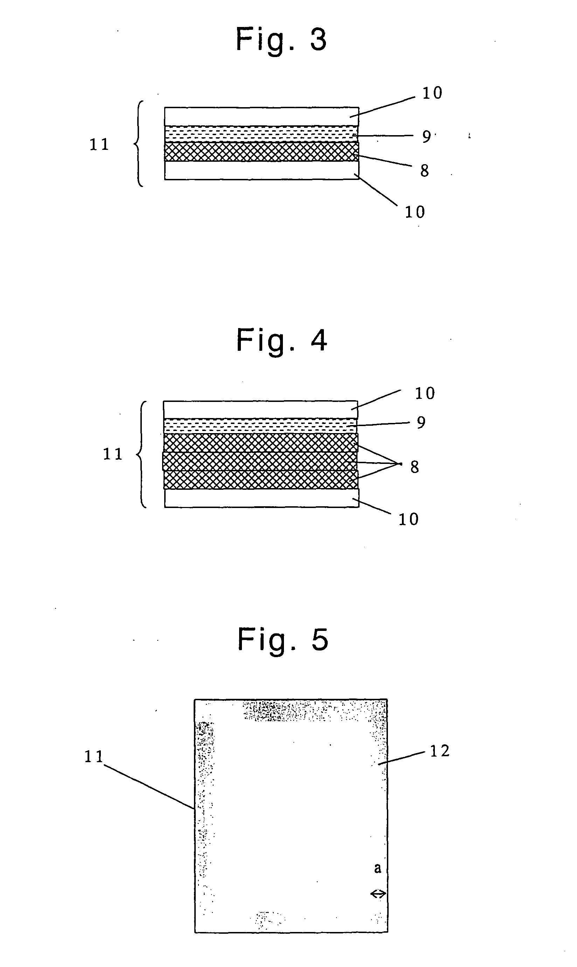

The cross-sectional views of composite filters which can be used in the present invention are shown in Figs. 3 and 4. The composite filter shown in Fig. 3 is obtained by laminating nonwoven fabric 10, prefilter 9, virus removal filter 8 and nonwoven fabric 10 in this order. In this composite filter, nonwoven fabric 10 is provided on both the top side and the bottom side of the laminated filters, but it is not necessary to provide a nonwoven fabric on both the top and bottom sides of the laminated filters. Further, there is no particular limitation with respect to the number of filters and nonwoven fabrics laminated, and multiple prefilters and virus removal filters can be laminated. For example, in the composite filter shown in Fig. 4, three virus removal filters are laminated. When especially high virus removal capability is required of the composite filter, high efficacy of virus removal can be attained by laminating multiple virus removal filters together with a prefilter and a nonwoven fabric.

After laminating the filters and the nonwoven fabric in the above-mentioned manner, it is advantageous that the peripheral portions of the filters and nonwoven fabric of the resultant laminate are adhered to each other so as to form a peripheral adhesion area having a predetermined width, because the resultant composite filter having a peripheral adhesion area can be handled easily during the subsequent processing of the composite filter. An example of such a composite filter is shown in Fig. 5. Fig. 5 shows a plan view of composite filter 11 having adhesion area 12. There is no particular limitation with respect to the width "a" of the adhesion area. However, when a filter bag is produced using a composite filter, although the width of the adhesion area varies depending on the size of the filter bag, the width of the adhesion area is preferably 1 to 20 mm, more preferably 2 to 10 mm. As preferred examples of methods for forming the adhesion area, there can be mentioned heat adhesion, ultrasonic adhesion, high frequency adhesion, and adhesion by an adhesive, such as an epoxy resin, a formaldehyde resin, an unsaturated polyester resin, a polyurethane resin, a silicon-containing resin, a polyvinyl acetate resin and a polyvinyl alcohol resin.

It is also possible to prepare a unified structure comprising a virus removal filter and a prefilter by a co-extrusion method. The co-extrusion is a technique in which different raw materials for preparing a membrane are extruded simultaneously through a single die of an extruder. When a filter is produced using the co-extrusion method, in the case of the heat-melt process, the average pore diameter of the filter can be controlled by changing the mixing ratio of a plasticizer and the raw materials of the filter, and in the case of the wet process, the average pore diameter of the filter can be controlled by changing the mixing ratio of a solvent and the raw materials of the filter.

Further, the strength of the composite filter used in the present invention can be improved by further providing a net or a porous material on the top side or the bottom side of the composite filter.

In the present invention, there is no particular limitation with respect to the material of a virus removal membrane (a virus removal filter, or a prefilter contained in the composite filter) which constitutes at least a part of the separation membrane (b). However, when the virus-containing suspension to be filtered is plasma, it is preferred that the inside surfaces of pores which contact the plasma is hydrophilic and that the inside surfaces of pores have a surface composition which is free from the adsorption of proteins contained in the plasma. Examples of materials which can be used to prepare a virus removal membrane having the above -mentioned properties include a hydrophilic polyfluoro vinylidene, a hydrophilic polysulfone, a polyacrylonitrile, a cellulose, a regenerated cellulose, a cellulose acetate, a cross-linked polyvinyl alcohol, an ethylene vinyl alcohol copolymer and a mixture thereof. These materials can be used to produce a porous membrane by a dry process or a wet process.

In the present invention, it is preferred that the virus removal membrane (a virus removal filter, or a prefilter contained in the composite filter) is a hydrophilic porous membrane obtained by an addition of a hydrophilic compound onto the surface of a porous membrane, and it is more preferred that the addition of a hydrophilic compound is a graft polymerization reaction of a hydrophilic monomer. Such a hydrophilic porous membrane is obtained by producing a porous membrane and adding a hydrophilic compound onto the surface (including the inner surfaces of the pores) of the produced porous membrane. With respect to the raw materials for the porous membrane before adding a hydrophilic compound onto the surface thereof, there can be mentioned polyolefins, such as a polyethylene, a polypropylene and a polybutylene; a copolymer of an olefin and a halogenated olefin; a polyfluoro vinylidene; a polychloro vinylidene; a cellulose acetate and a mixture thereof. The average pore diameter of the porous membrane before the addition of a hydrophilic compound is preferably in the range of from 1 to 100 nm, more preferably from 10 to 80 nm, most preferably from 20 to 70 nm. The thickness of the porous membrane is preferably in the range of from 5 to 500 μm, more preferably from 10 to 200 μm. The porosity of the porous membrane is preferably in the range of from 5 to 80 %, more preferably from 10 to 40 %.

Examples of hydrophilic compounds used for the addition onto the surface of the above-mentioned porous membrane include hydrophilic monomers, such as acrylic acid, methacrylic acid, an ester of acrylic acid or methacrylic acid with a polyhydric alcohol, and a mixture thereof. Specific examples of the ester of acrylic acid or methacrylic acid with a polyhydric alcohol include glycidyl methacrylate, hydroxypropyl acrylate, hydroxyethyl acrylate, hydroxyethyl methacrylate, methoxyethyl acrylate, methyl methacrylate, ethyl acrylate, ethylhexyl acrylate and phenoxyethyl acrylate. Further, polymers having a cross-linkable group (such as a vinyl group and an allyl group) in the main chain or side chain thereof may be used as the hydrophilic compound. Specific examples of such polymers include a polyethylene oxide having a vinyl group or an allyl group, a polygricydol and a polyvinylpyrrolidone. Copolymers containing monomer units selected from the monomer units constituting the above-mentioned polymers can also be used as the hydrophilic compound. As a method for adding the hydrophilic compound onto the surface of a porous membrane, most suitable is a method in which a porous membrane is subjected to an ionization radiation by an electron beam or gamma ray to thereby generate radicals, and the resultant porous membrane carrying radicals is placed in contact with a hydrophilic compound in the form of a liquid or gas to thereby effect an addition reaction (i.e., graft polymerization reaction). As an alternative method for adding a hydrophilic compound onto the surface of a porous membrane, there can be mentioned a method in which the surface of a porous membrane is coated with the above-mentioned polymer having a cross-linkable group (such as a vinyl group or an allyl group) in the main chain or side chain thereof, and the resultant polymer coating is cross-linked by heat, radiation or a cross-linking agent; and a method in which the surface of a porous membrane is coated with a hydrophilic polymer, such as a polyvinyl alcohol or an ethylenevinyl alcohol copolymer. These methods are recommendable from the viewpoint of ease in operation. Further, in the case where a method is used in which a hydrophilic compound is added onto a porous membrane by the addition reaction, there may be performed an operation in which a diacrylate compound, such as polyethylene glycol diacrylate, is mixed with the hydrophilic compound before the addition reaction with a porous membrane, and after the addition reaction, the resultant addition-modified porous membrane is subjected to a cross -linking reaction.

With respect to the hydrophilicity of the materials of the virus removal filter or prefilter, it is preferred that the contact angle as measured with respect to a flat membrane is in the range of from 0 to 140°, more advantageously from 0 to 120°, still more advantageously from 0 to 90°. When the contact angle is 140° or less, it becomes possible to obtain high liquid perviousness. In the present invention, the contact angle is a value as measured with respect to a porous flat membrane by using an automated contact angle meter (DCA-VM model, manufactured and sold by Kyowa Interface Science Co., Ltd., Japan).

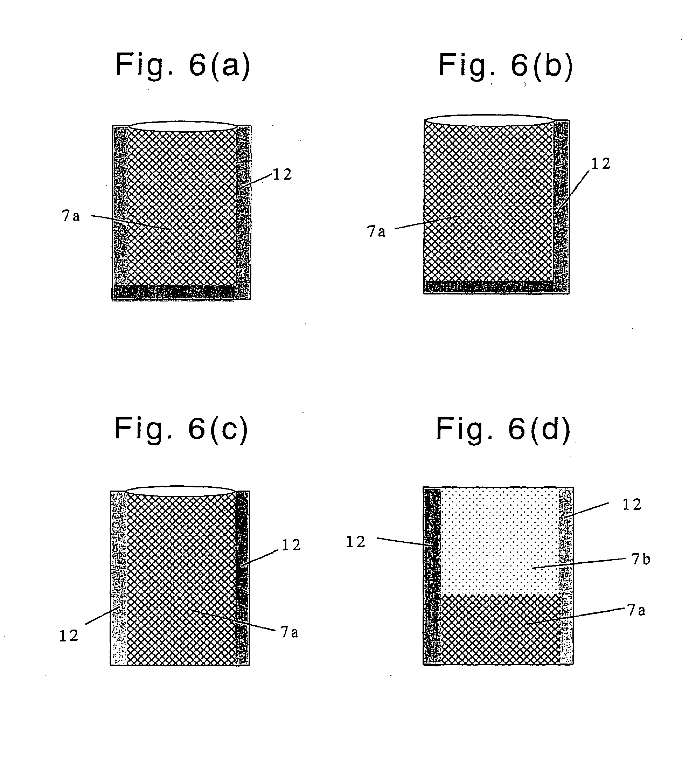

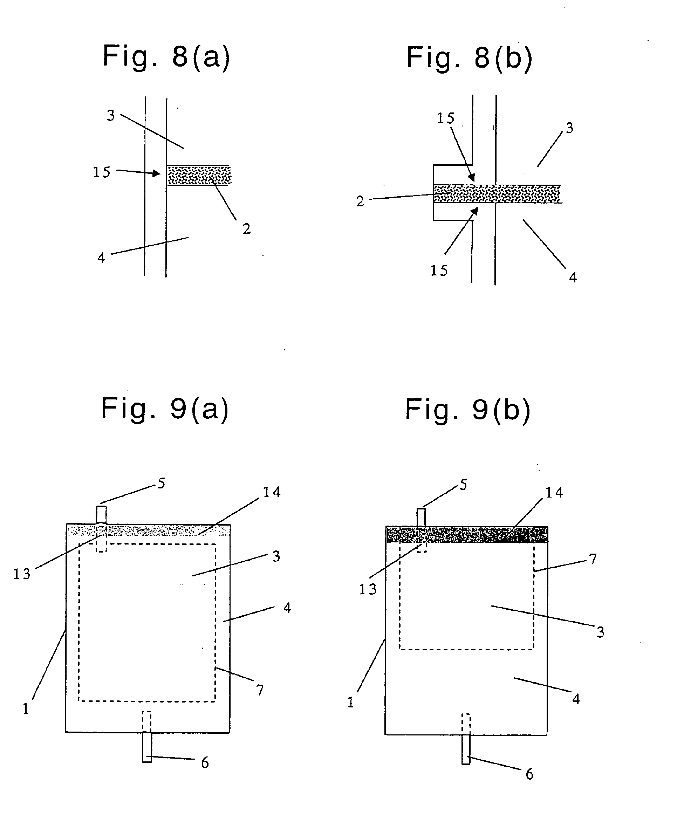

Next, an explanation is made with respect to a filter bag used in a virus removal bag having a separation membrane (b) in the form of a bag. As explained above, at least a part of the surrounding wall of the filter bag is made of a virus removal membrane for removing viruses from a virus-containing suspension by filtration. The filter bag used in the present invention is obtained by fabricating a virus removal membrane (such as a virus removal filter or the above-mentioned composite filter) into a bag, or by fabricating into a bag a composite material which is prepared by connecting a virus removal filter to a sheet made of another material. Figs. 6(a) to 6(c) show schematic views of a filter bag prepared by fabricating virus removal membrane(s) into a bag. Fig. 6(a) shows a filter bag obtained by putting two virus removal membranes on top of each other to form a laminate structure having four sides and, then, sealing three of the four sides of the laminate structure, thereby fabricating the two virus removal membranes into a bag. Figs. 6(b) and 6(c) show a filter bag obtained by folding one virus removal membrane in two (specifically, the left peripheral edge of the filter bag shown in Fig. 6(b) and the bottom peripheral edge of the filter bag shown in Fig. 6(c) are the folds) to form a laminate structure having four sides (comprising one folded side and three non-folded sides) and, then, sealing two non -folded sides of the four sides of the laminate structure, thereby fabricating the virus removal membrane into a bag. The width of the sealed portion may vary depending on the size of the filter bag, but in general, it is preferably in the range of from 1 to 20 mm, more preferably from 2 to 10 mm. The opening of the filter bag may be worked on to form a structure wherein the opening of the filter bag sealedly communicates with the inlet of the virus removal bag. For example, the opening of the filter bag may be worked on to form a structure in which the opening is sealedly closed so as to have a tube inserted therein, wherein the opening of the filter bag and the opening of a pouchy casing (a) may be overlapped and sealedly closed together along the entire length of the opening of the pouchy casing (a) (i.e., along the entire upper peripheral portion of the pouchy casing (a)) (see Fig. 9).

As explained above, at least a part of the membranous, overall surrounding wall of the filter bag used in the present invention is made of a virus removal membrane. Specifically, at least 10 %, preferably at least 20 % of the membranous, overall surrounding wall of the filter bag which contacts the virus-containing suspension is made of a virus removal membrane. Therefore, a part of the membranous, overall surrounding wall of the filter bag which is not made of a virus removal membrane, if any, can be made of a sheet which is impervious to a virus-containing suspension. Examples of materials used for producing such sheets which are impervious to a virus-containing suspension include resins, such as a polyvinyl chloride, a polypropylene, a polyethylene, a polyethylene terephthalate, a nylon, a polyurethane and a styrene-isobutylene-styrene copolymer. It is preferred that the resin is a soft material containing a plasticizer and the like. Fig. 6(d) shows a filter bag in which a part of its overall surrounding wall is made of virus removal membrane 7a and the remaining part of the overall surrounding wall is made of sheet 7b which is impervious to a virus -containing suspension. In this filter bag, virus removal membrane 7a and sheet 7b which is impervious to a virus-containing suspension are adhered to each other.

It is preferred that the virus removal bag of the present invention is flexible. In the present invention, the expression "the virus removal bag is flexible" means that most of the components constituting the virus removal bag (namely a pouchy casing (a), a separation membrane (b), a filter bag, and a tube used as an inlet or an outlet) are made of a flexible material. Specifically, for maintaining the overall strength of the virus removal bag, parts of the virus removal bag (e.g., parts for holding a tube used to connect the filter bag to another functional bag) and a joint formed between a filter bag and a pouchy casing (a) can be made of a non-flexible material (such as a hard plastic). However, in the present invention, it is recommended that the pouchy casing (a) and the filter bag are made of a flexible material. When a centrifugal force is applied to a virus removal bag which is connected to other functional bags and the like, a flexible virus removal bag is capable of preventing other functional bags from being damaged. As examples of flexible materials used for producing a virus removal bag, there can be mentioned soft materials obtained by adding a plasticizer and the like to polymeric materials, such as a polyvinyl chloride, a polyurethane, a polypropylene, a polyethylene, an ethylene-vinyl acetate copolymer, a polyethylene terephthalate and a nylon, and an especially preferred material is a thermoplastic elastomer containing a soft polyvinyl chloride, a polyurethane or an ethylene-vinyl acetate copolymer as a main component thereof. Herein, a flexible material is a material having a flexibility which enables the material to be bent by hand.

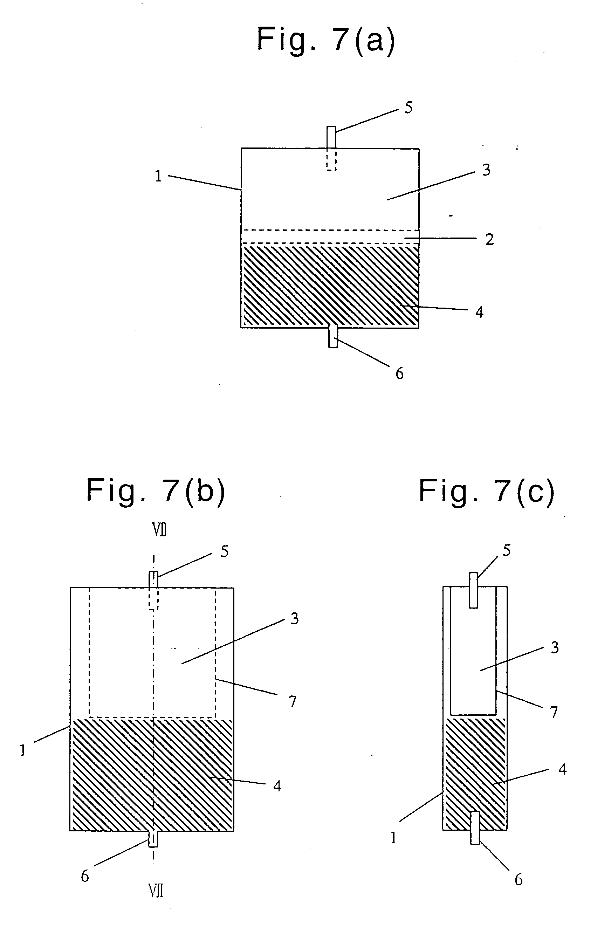

In the virus removal bag of the present invention, it is preferred that the second compartment (d) has a volume sufficient to collect all of the filtrate obtained by filtering the virus-containing suspension through the virus removal membrane. Use of a virus removal bag having such second compartment (d) is advantageous for the following reason. In the case of the use of such second compartment (d), when a virus -containing suspension is introduced into the first compartment (c) of the virus removal bag and the virus -containing suspension is filtered through the virus removal membrane (e.g., using a centrifugal force), all of the resultant filtrate which is the virus-removed suspension is collected in the second compartment (d). Therefore, there is no need to provide an additional bag for collecting the filtrate, wherein the additional bag is separately connected through a liquid transfer pipe and the like to the virus removal bag. In the present invention, the expression "the second compartment (d) has a volume sufficient to collect all of the filtrate obtained by filtering the virus-containing suspension through the virus removal membrane" means that the volume of the second compartment (d) is the same as or larger than the volume of the virus -containing suspension to be filtered. Fig. 7(a) shows a schematic view of a virus removal bag containing separation membrane (b) in the form of a sheet, and the portion of the virus removal bag used to determine the volume of the second compartment (d) is shown by hatching. Further, Figs. 7(b) and 7(c) show schematic views of a virus removal bag having separation membrane (b) in the form of a membranous, overall surrounding wall of a filter bag, and the portion of the virus removal bag used to determine the volume of the second compartment (d) is shown by hatching. The volume of the second compartment (d) of the above-mentioned two types of virus removal bags is determined as follows. The point of contact between an outlet and a second compartment of a virus removal bag is pinched using a pinchcock and pure water is added to the second compartment. The resultant virus removal bag containing pure water is caused to stand still in the upright position or alternatively, the resultant virus removal bag is hung down in the upright position, followed by measuring the volume of pure water contained in the portion of the second compartment (d) which corresponds to the hatched portion shown in Fig. 7. The measured value is used as the volume of the second compartment (d).

When the virus removal bag of the present invention is used for the treatment of blood or plasma, in view of the standard blood volume per donation, it is preferred that the volume of a first compartment (c) of the virus removal bag is 100 to 600 $cm^3$, more advantageously 150 to 250 $cm^3$. It is preferred that the volume of a second compartment (d) which is sufficient for collecting all of the filtrate obtained by filtering the virus-containing suspension through the virus removal membrane is 100 to 800 $cm^3$, more advantageously 150 to 400 $cm^3$.

In a virus removal bag having a separation membrane (b) in the form of a sheet, the separation membrane (b) is securely held in the pouchy casing (a) and partitions the internal space of the pouchy casing (a) into a first compartment (c) communicating with the inlet and a second compartment (d) communicating with the outlet. There is no particular limitation with respect to the method for securely holding the separation membrane (b) in the pouchy casing (a) and, for example, there can be mentioned heat adhesion, ultrasonic adhesion, high frequency adhesion, and adhesion by an adhesive, such as an epoxy resin, a formaldehyde resin, an unsaturated polyester resin, a polyurethane resin, a silicon-containing resin, a polyvinyl acetate resin or a polyvinyl alcohol resin. Fig. 8 shows schematic views explaining the joint between a separation membrane (b) in the form of a sheet and a pouchy casing (a) in the virus removal bag of the present invention. Fig. 8(a) shows a joint formed between a separation membrane (b) in the form of a sheet and an inner wall of a pouchy casing (a). A virus removal bag having such a structure can be produced by separately providing a pouchy casing (a) and a separation membrane (b) in the form of a sheet and fixing the separation membrane (b) to the pouchy casing (a) by an adhesive. Fig. 8(b) shows a joint formed by adhering peripheral portions of at least two sheets (forming a pouchy casing (a)) to each other through a peripheral portion of a separation membrane (b) in the form of a sheet. A virus removal bag having such a structure can be produced by providing at least two sheets for producing a pouchy casing (a), and adhering peripheral portions of the at least two sheets to each other through a peripheral portion of a separation membrane (b) in the form of a sheet. For example, a virus removal bag can be produced in accordance with the method used in Example 7 of the present specification. Specifically, two casing halves (first and second casing halves) each having a fixation collar at the periphery thereof are provided (wherein the first casing half has an inlet and the second casing half has an outlet), and the fixation collars of the first and second casing halves are adhered to each other through a peripheral portion of the separation membrane (b), to thereby obtain a virus removal bag. With respect to the joint formed between a separation membrane (b) in the form of a sheet and a pouchy casing (a), the joint shown in Fig. 8(b) is preferred because such joint exhibits high adhesion strength.

In a virus removal bag having a separation membrane (b) in the form of a bag (filter bag), the filter bag is securely held in a pouchy casing (a), such that the membranous, overall surrounding wall of the filter bag partitions the internal space of the pouchy casing (a) into a first compartment (c) which is the internal space of the filter bag communicating with the inlet and a second compartment (d) which is the internal space of the pouchy casing (a), exclusive of a filter bag portion, communicating with the outlet. There is no particular limitation with respect to the method for securely holding the filter bag in the pouchy casing (a) and, as in the case of a separation membrane (b) in the form of a sheet, there can be used heat adhesion, ultrasonic adhesion, high frequency adhesion, and adhesion by an adhesive. A filter bag and a pouchy casing (a) can be connected to each other by, for example, the method shown in Fig. 9. In Fig. 9(a), filter bag 7 is connected to pouchy casing 1 through tube 13 used as inlet 5. In this virus removal bag, the only point of connection between filter bag 7 and pouchy casing 1 is present at tube 13 for introducing a virus-containing suspension. Fig. 9(b) shows a virus removal bag in which filter bag 7 and pouchy casing 1 are connected to each other along the entire upper peripheral portion of pouchy casing 1. In such a virus removal bag having a large joint formed between a pouchy casing and a filter bag, the joint is very strong and, thus, this virus removal bag is advantageous for use in filtration by centrifugation which causes large stress on the virus removal bag. Sealed portion 14 of pouchy casing 1 is shown in both Figs. 9(a) and 9(b). When tube 13 (used as inlet 5) is sandwiched between two walls of a pouchy casing and the resultant sandwich structure is subjected to heat sealing, it is important that the temperature, pressure and time used for the heat sealing should be controlled so as to prevent the tube from being completely flattened.

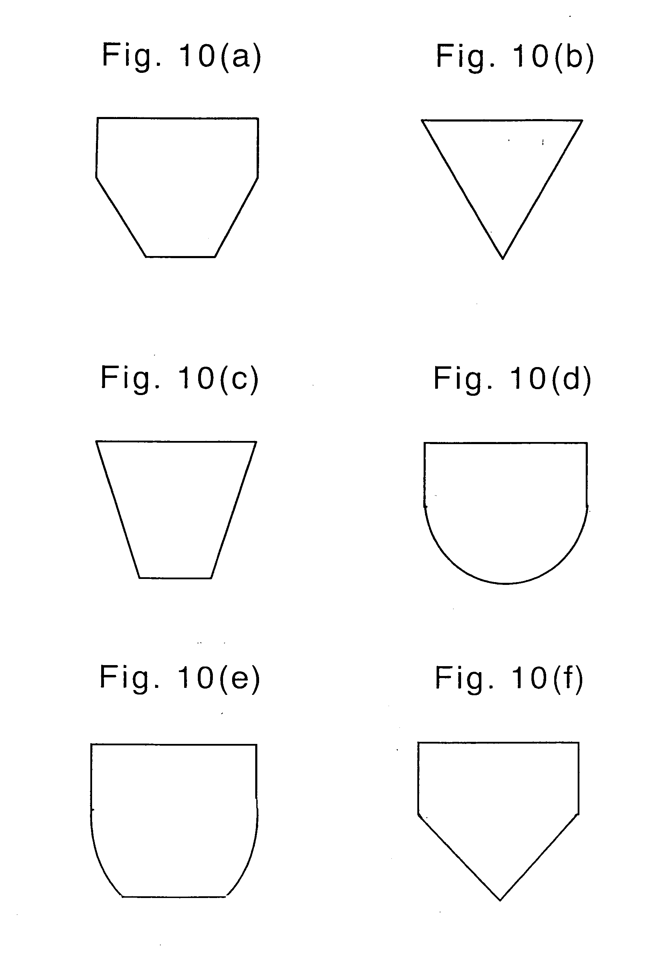

In the present invention, it is preferred that the filter bag is tapered toward the forward end of the filter bag as viewed in a flow direction of the virus -containing suspension in the virus removal bag, wherein the tapering begins at the backward end of the filter bag or at a portion during the course toward the forward end of the filter bag. In other words, it is preferred that the filter bag used in the present invention has a tapered shape like a bag which is squeezed at the bottom thereof. Examples of filter bags which are tapered toward the forward end of the filter bag are shown in Fig. 10. With respect to the filter bag used in the present invention, like the filter bags shown in Figs. 10(b) and 10(c), the tapering may begin at the backward end of the filter bag. Alternatively, like the filter bags shown in Figs. 10(a), 10 (d), 10(e) and 10(f), the tapering may begin at a portion during the course toward the forward end of the filter bag. For example, like the filter bags shown in Figs. 10(d) and 10(e), the tapering of the filter bag may be such that the filter bag has a curved contour. For producing a tapered filter bag, it is recommendable to use, for example, a method in which a virus removal membrane or a membrane having at least a part thereof made of a virus removal membrane is cut so as to obtain two membrane sheets of the same shape which is one of the shapes shown in Figs. 10(a) to 10(f) and, then, the mutually corresponding peripheral portions of the obtained membrane sheets are bonded to each other by heat sealing or with an adhesive, to thereby obtain a tapered filter bag.

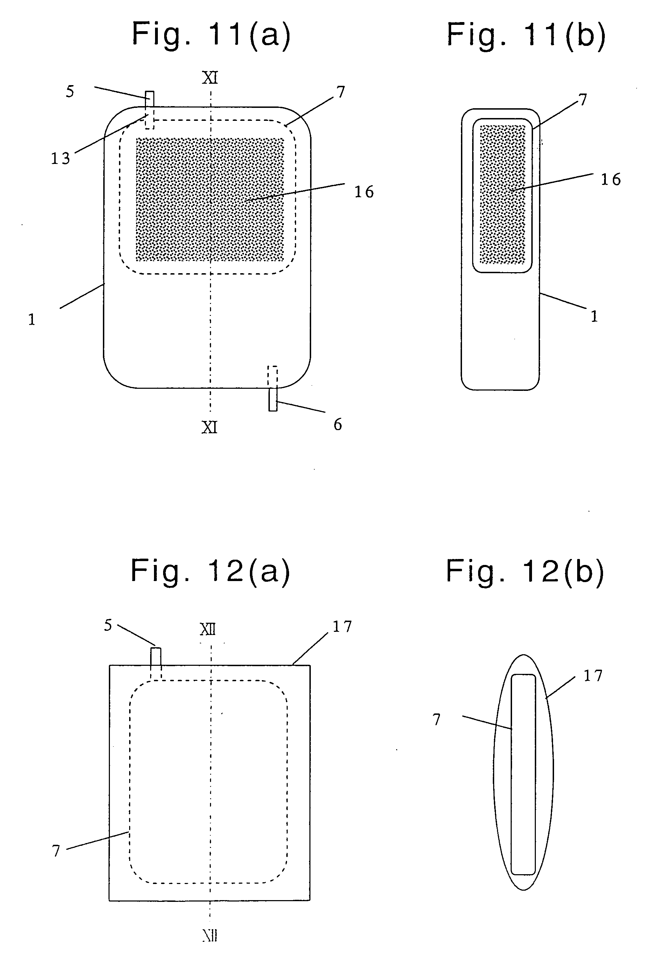

In the present invention, it is preferred that a first compartment (c) of the virus removal bag contains a spongy adsorber. An example of a virus removal bag having a filter bag (i.e., a first compartment (c)) containing a spongy adsorber is shown in Fig. 11. When the virus removal bag contains a spongy adsorber, lipids and other impurities contained in a virus -containing suspension are adsorbed by the spongy adsorber and this leads to an improvement in the filtration efficiency. Preferred examples of spongy adsorbers include a urethane foam and a melamine foam, each of which not only can be easily compressed by applying pressure thereto, but also can stand autoclaving sterilization. There is no particular limitation with respect to the size of the spongy adsorber, but it is preferred that the volume of the spongy adsorber is in the range of from 30 to 90 % of the volume of a first compartment (c).

In the case where a virus-containing suspension is filtered using a virus removal bag containing a filter bag, when the filtration of the virus-containing suspension through the virus removal membrane is promoted by applying a pressure to the virus-containing suspension in the first compartment (c), for assuring a passage for a filtrate which is a virus-removed suspension, it is preferred that the filter bag is enclosed in a spacer. A spacer used in the present invention is a resin net made of a polypropylene, a polyethylene, polyethylene terephthalate or the like, and its mesh size is preferably in the range of from 0.5 to 20 mm. The spacer is a bag which is large enough to enclose a filter bag therein, and it is preferred that the spacer containing a filter bag is securely held in a pouchy casing (a). An example of a filter bag enclosed in a spacer is shown in Fig. 12. In Fig. 12, spacer 17 covers filter bag 7, and it is preferred that the filter bag is covered by the spacer so that the end of tube 13 (used as inlet 5) connected to the filter bag is positioned outside of the spacer.

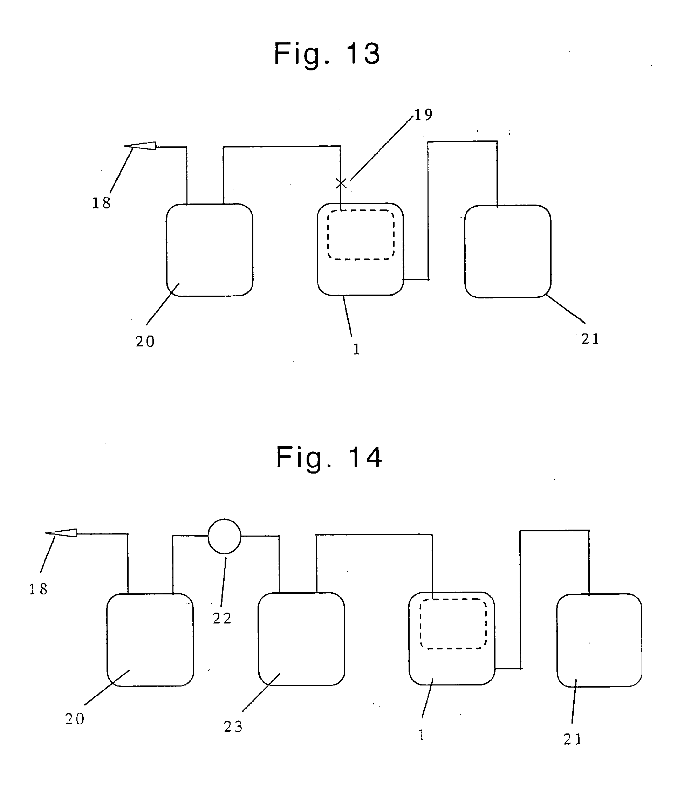

It is preferred that the virus removal bag of the present invention is aseptically and fluid-tightly connected to at least one functional bag which has a function other than a virus removal function, thereby providing a closed, multi-bag virus removal system. In the present invention, a "closed, multi-bag virus removal system" is a closed system having a structure in which conventional functional bag(s) used in the field of blood treatment (specifically, blood bag (s) used for collecting blood components and the like) is (or are) aseptically and fluid-tightly connected to the above -mentioned virus removal bag, wherein the system can be used for removing viruses from a virus-containing suspension. As specific examples of closed, multi-bag virus removal systems, there can be mentioned the virus removal systems shown in Figs. 13 to 22 which are used for preparing a virus-removed plasma.

The multi-bag virus removal system for preparing a virus-removed plasma contains a blood bag which is a functional bag. A "blood bag" is a functional bag for collecting blood components. In the present invention, the term "blood component" encompasses whole blood, plasma, erythrocytes, leukocytes, platelets and a mixture thereof (e. g., buffy coat which is a mixture of leukocytes and platelets). In general, after collecting blood (whole blood), the collected blood is separated into blood components by centrifugation or the like before storage. Therefore, in a multi-bag system used for treating blood, bags (such as a blood bag for whole blood, a blood bag for plasma and a blood bag for blood cells) and functional units (such as a leukocyte removal filter unit mentioned below) are preassembled to thereby form a system comprising multiple bags and units. Further, the bags of a multi-bag system may be provided so as to contain a physiological solution (such as a physiological saline) or an additive (such as a blood anticoagulant). Alternatively, it is also possible to provide a separate bag containing an additive and connect the bag containing the additive to the multi-bag system. When the filter bag of the virus removal bag contains a spongy adsorber mentioned above, air contained in the spongy adsorber must be removed therefrom before introducing plasma into the filter bag and, thus, a separate bag for collecting air contained in the spongy adsorber is necessary. Such a bag for removing air from the virus removal bag may be connected to the multi-bag system. In addition, when it is intended to complete the filtration by sending air into the virus removal bag to thereby pressurize the bag and promote the filtration of liquid remaining therein, a bag containing gas for pressurization may be connected to the multi-bag system. It is preferred that the above-mentioned bags are connected to each other through transfer pipes which are flexible tubes made of a flexible material which is the same as that used for producing the bags.

In a "closed, multi-bag virus removal system" containing the virus removal bag of the present invention and the below-mentioned "closed, virus removal system" used in the methods of the present invention, the virus removal bag is aseptically and fluid-tightly connected to other functional bags and filter units. In the present invention, the expression "aseptically and fluid -tightly connected" means a situation in which the virus removal bag is connected through a transfer pipe (such as a tube) to a functional bag or the like before introducing a liquid thereinto, thereby forming an assembly, and the resultant assembly is hermetically sealed to thereby obtain a closed system in which the inside thereof maintains aseptic conditions. Under such conditions, the inside of the functional bag remains unexposed to open air and it becomes possible to prevent bacteria and viruses from contaminating the liquid being treated using the system. However, for enabling withdrawal of liquid from the system or addition of liquid into the system as needed, a part of the system may be provided with a cannula or an openable means with a cock. In addition, a multi-bag system in which the proliferation of bacteria and viruses is inhibited can be obtained by connecting a functional bag and a virus removal bag to assemble a multi-bag system, and subjecting the assembled multi-bag system to a sterilization treatment using heat, steam or radiation to kill bacteria and viruses present inside of the multi-bag system. In a multi-bag virus removal system, in most cases a virus removal bag is connected to several functional bags and, therefore, it is preferred that all bags contained in the multi-bag virus removal system are connected to each other before subjecting the system to a sterilization treatment. For example, when the sterilization is performed using heat, it is preferred that the sterilization temperature is in the range of from 90 °C to 150 °C, more advantageously from 100 °C to 130 °C, and that the sterilization time is in the range of from 5 to 120 minutes, more advantageously from 20 to 60 minutes. In the multi-bag system, the number of virus removal bag connected to the functional bags is not limited to one, and several virus removal bags may be connected in accordance with the amount of the virus-containing suspension to be treated with the multi-bag system.

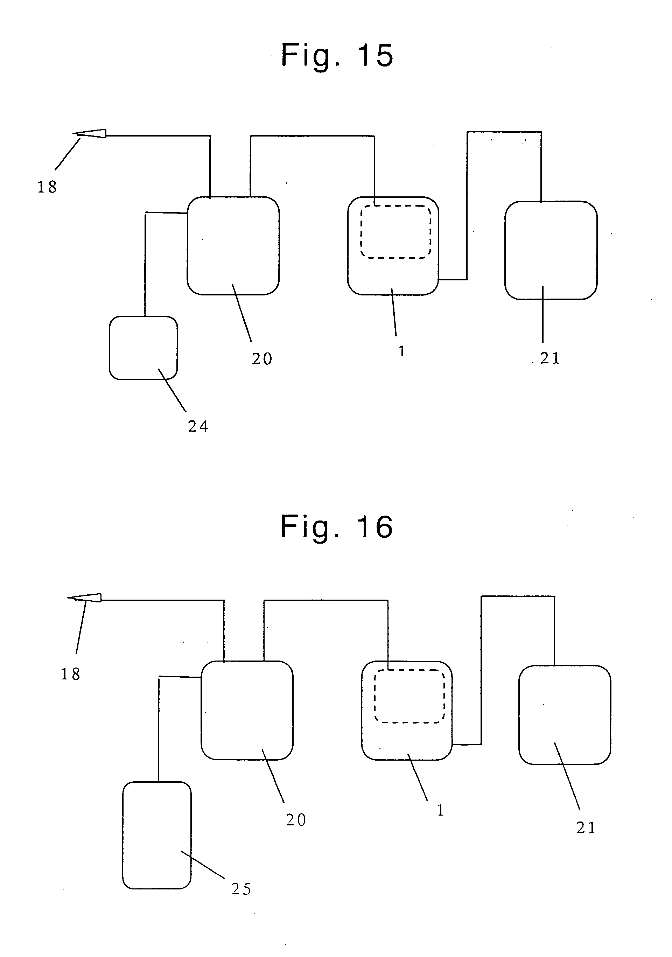

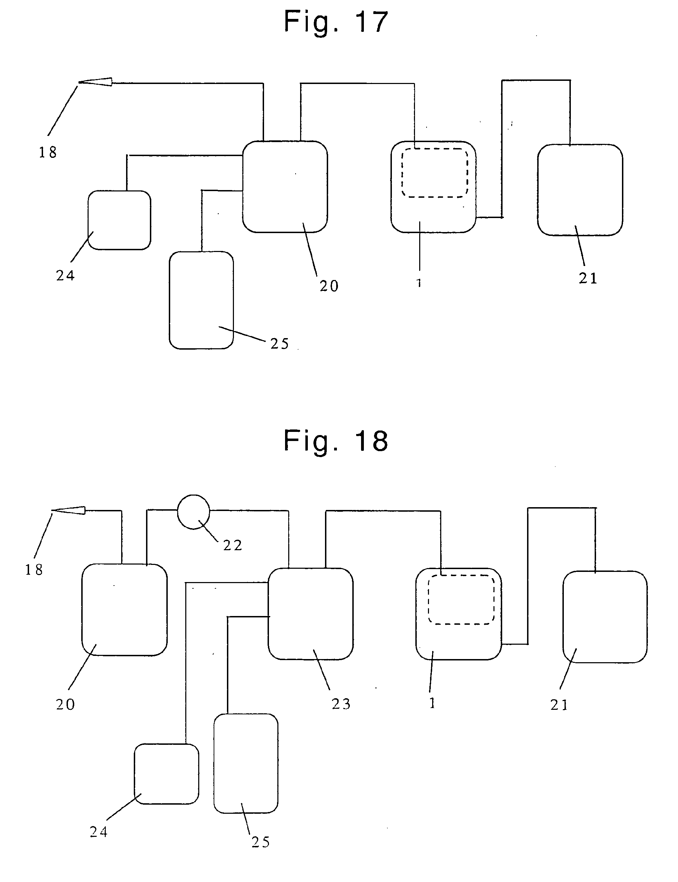

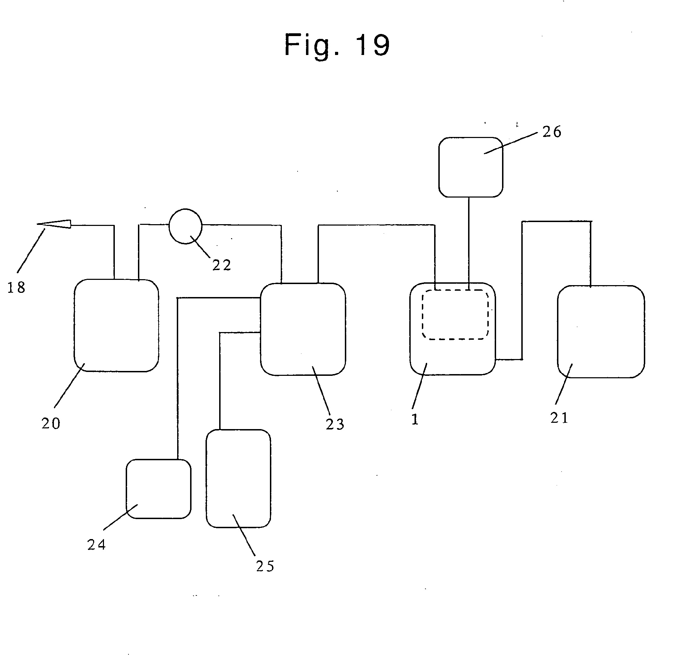

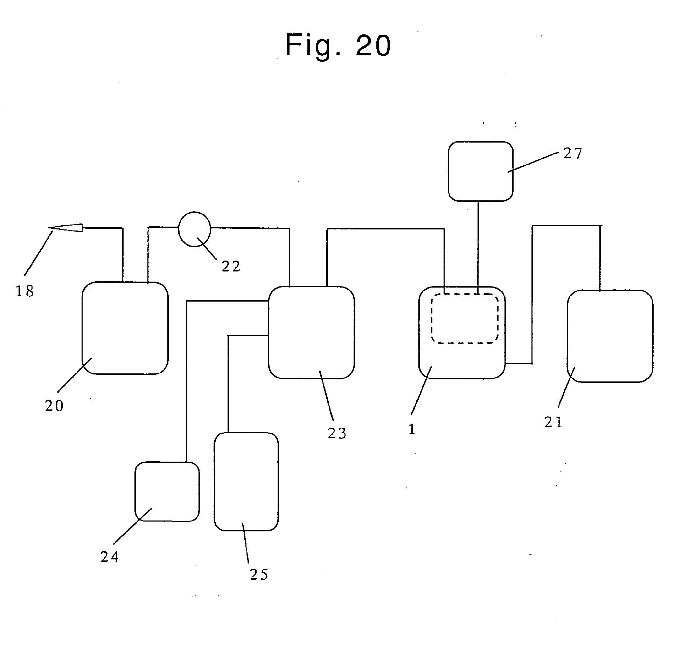

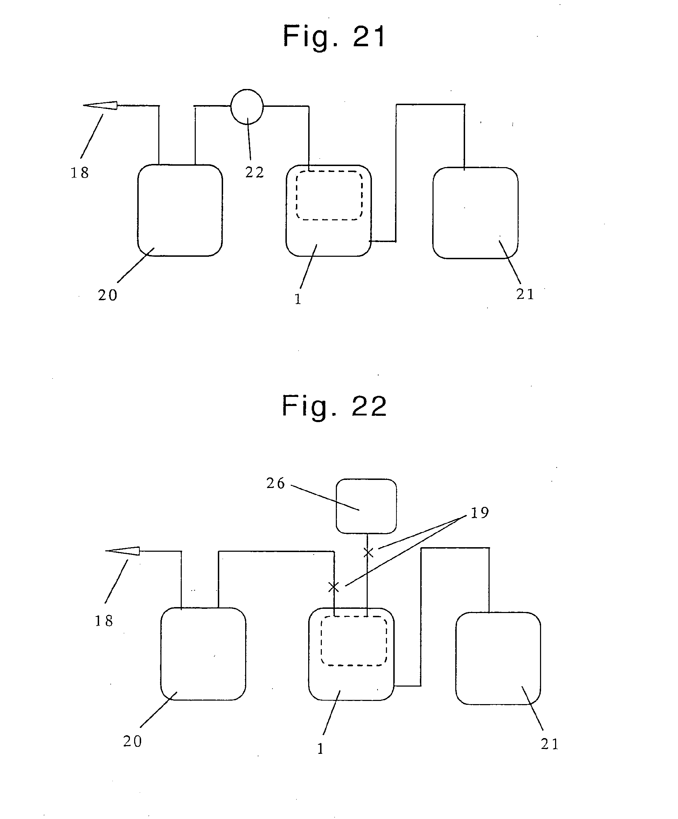

Figs. 13 to 22 each show a schematic view explaining an example of a closed, virus removal system comprising the virus removal bag of the present invention which is for obtaining a virus-removed plasma. Each of the systems shown in Figs. 13 to 22 comprises a blood collector means comprising cannula 18 and blood bag 20 for whole blood; a virus removal bag having pouchy casing 1; and a plasma recovery means comprising blood bag 21 for filtered plasma. In the drawings, the lines connecting a cannula, bags and the like are liquid transfer pipes. Fig. 13 shows a simple system comprised of cannula 18, blood bag 20 for whole blood, a virus removal bag having pouchy casing 1 and blood bag 21 for filtered plasma. Fig. 14 shows a system obtained by adding leukocyte removal unit 22 and blood bag 23 for leukocyte-removed blood to the system shown in Fig. 13. Fig. 15 shows a system obtained by adding blood bag 24 for buffy coat to the system shown in Fig. 13. Fig. 16 shows a system obtained by adding blood bag 25 containing additives and the like (such as an anticoagulant) to the system shown in Fig. 13. Fig. 17 shows a system obtained by adding blood bag 25 containing additives and the like (such as an anticoagulant) and blood bag 24 for buffy coat to the system shown in Fig. 13. Fig. 18 shows a system obtained by adding blood bag 25 containing additives and the like (such as an anticoagulant) and blood bag 24 for buffy coat to the system shown in Fig. 14. Fig. 19 shows a system obtained by adding bag 26 for removing air from a virus removal bag to the system shown in Fig. 18. Fig. 20 shows a system obtained by adding bag 27 containing gas for pressurization to the system shown in Fig. 18. Fig. 21 shows a system obtained by adding leukocyte removal unit 22 to the system shown in Fig. 13, wherein leukocyte removal unit 22 is positioned immediately before the virus removal bag having pouchy casing 1. Fig. 22 shows a system obtained by adding bag 26 for removing air from a virus removal bag to the system shown in Fig. 13. Further, portion 19 of a liquid transfer pipe to be melt-sealed and cut is representatively shown in Figs. 13 and 22, and portion 19 shows which part of the system is preferably cut away (for example, by melt -sealing/cutting) after introducing plasma into the virus removal bag.

In another embodiment of the present invention, the present invention provides a method for removing viruses from a virus-containing suspension, comprising:

(1) providing at least one virus removal bag;
(2) introducing a virus-containing suspension to the virus removal bag through the inlet, to receive the virus-containing suspension in the first compartment (c);

(3) filtering the virus-containing suspension through the virus removal membrane, to thereby remove viruses from the suspension;

(4) collecting a filtrate which is a virus -removed suspension in the second compartment (d); and

(5) withdrawing the virus-removed suspension through the outlet.

In the method of the present invention for removing viruses from a virus-containing suspension (hereinafter, frequently referred to as the "virus removal method of the present invention"), the virus removal bag of the present invention as disclosed above is used. The virus removal bag used may contain any of a separation membrane (b) in the form of a sheet and a separation membrane (b) in the form of a bag (i.e., a filter bag).

In step (2) of the virus removal method of the present invention, a virus-containing suspension is introduced to the virus removal bag through the inlet, to thereby receive the virus-containing suspension in the first compartment (c). In the subsequent step (3), the virus-containing suspension is filtered through the virus removal membrane, to thereby remove viruses from the suspension and, in step (4), a filtrate which is a virus-removed suspension is collected in the second compartment (d). There is no particular limitation with respect to the method for filtering the virus -containing suspension. However, it is preferred that the filtration of the virus-containing suspension through the virus removal membrane is promoted by applying a centrifugal force or a pressure to the virus -containing suspension in the first compartment (c).

When a centrifugal force is applied to the virus -containing suspension in the first compartment (c), the centrifugal force is applied so as to cause the virus-containing suspension in the first compartment (c) to move through the virus removal membrane into the second compartment (d), thereby forming a filtrate. In other words, the virus removal bag is rotated so that the centrifugal force is exerted in a flow direction of the virus-containing suspension. The magnitude of the centrifugal force applied to the first compartment (c) may be selected depending on the size of a centrifuge employed and the strength of the virus removal bag. However, it is preferred that the magnitude of the centrifugal force is in the range of from 5 G to 70,000 G. When functional bags and the like are connected to the virus removal bag, if desired from the viewpoint of preventing bacterial contamination from the outside, the virus removal bag may be subjected to centrifugation without cutting off the functional bags and the like therefrom (i.e., the functional bags and the like which are connected to the virus removal bag may be subjected to centrifugation together with the virus removal bag). On the other hand, when it is difficult for the virus removal bag and other functional bags and the like to be accommodated in an appropriate place in the centrifuge, the tubes connecting the functional bags and the like to the virus removal bag can be cut and sealed by melt-seal/cutting to thereby remove unnecessary components connected to the virus removal bag. Then, the resultant virus removal bag having only necessary components (such as a functional bag) attached thereto may be subjected to centrifugation. It is preferred that the centrifugation is performed at a temperature in the range of from 0 to 40 °C, more advantageously from 5 to 35 °C. When the centrifugation is performed at a temperature in the above-mentioned range, even when the virus-containing suspension is plasma, the filtration can be performed at a high speed without causing serious denaturation of the virus-containing suspension.

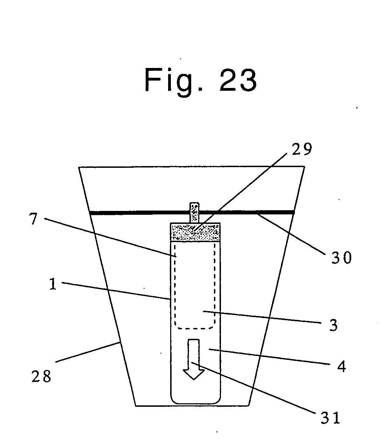

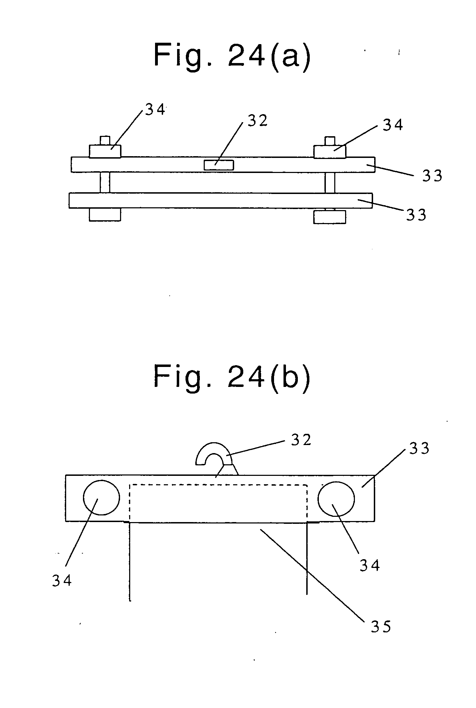

Fig. 23 shows a schematic view explaining an example of a virus removal bag placed in a centrifuge cup, and the virus removal bag shown in Fig. 23 has the same structure as that of the virus removal bag shown in Fig. 2. In Fig. 23, the upper portion of the virus removal bag having pouchy casing 1 is securely attached to securing hook 29, and securing hook 29 having the virus removal bag attached thereto is secured to securing bar 30 installed in a centrifugal cup of a centrifuge. By securing the virus removal bag to the centrifuge in such a manner, it becomes possible to suppress the distortion of the virus removal bag during centrifugation and perform the filtration satisfactorily. A white arrow shown in Fig. 23 indicates direction 31 of centrifugal force, and the filtration proceeds in this direction. Fig. 24 shows schematic views explaining an example of a securing hook used for securing a virus removal bag to a centrifuge cup. When using the securing hook shown in Fig. 24, upper portion 35 of the virus removal bag is sandwiched between holding plates 33 having hook 32, and the virus removal bag is secured to the securing hook with securing screw 33.

Fig. 25 shows schematic views explaining examples of methods for promoting the filtration by applying a centrifugal force to a virus removal bag, wherein Fig. 25(a) is a schematic view explaining a method for promoting the filtration by applying a centrifugal force to a virus removal bag having a separation membrane (b) in the form of a sheet. When the filtration is performed by the method shown in Fig. 25(a), the virus removal bag is securely attached to rotating element 36 and/or rotating element 37 as shown in the drawing (wherein the rotating element corresponds to a rotor of a centrifuge). When the rotating elements are rotated in the direction indicated by a black arrow in Fig. 25 (a), a centrifugal force is exerted in direction 31 which is indicated by a white arrow in the drawing, thereby promoting the filtration. The virus removal bag may be securely attached to either one or both of rotating elements 36 and 37. When the virus removal bag is securely attached to only one rotating element, the other rotating element (which is not attached to the virus removal bag) serves to prevent the virus removal bag from being detached from the rotating element during the rotation of the rotating element. On the other hand, when there is only small possibility of the detachment of the virus removal bag from the rotating element during the rotation of the rotating element, only one rotating element may be used. Figs. 25(b) and 25(c) each show a schematic view explaining a method for promoting the filtration by using only one rotating element. In the method shown in Fig. 25(b), the virus removal bag is securely attached to the outer side surface of the rotating element, and in the method shown in Fig. 25(c), the virus removal bag is securely attached to the inner side surface of the rotating element. The securing of the virus removal bag to the rotating element can be performed by any conventional method. For example, there can be mentioned a method in which a virus removal bag is screwed to a rotating element; a method in which a virus removal bag is secured to a rotating element by a belt which is wrapped around the rotating element so as to cover the virus removal bag; and a method in which a rotating element is provided with a recess for receiving a virus removal bag therein. Further, a virus removal bag can be wrapped around a rotating element. There is no particular limitation with respect to the length of the virus removal bag, and the length of the virus removal bag may be almost the same as the circumference of the rotating element. Fig. 25(d) shows a schematic view explaining a method for subjecting a virus removal bag to centrifugation, wherein the virus removal bag has almost the same length as the circumference of a rotating element. When the length of the virus removal bag is larger than the circumference of the rotating element, the virus removal bag may be attached to the rotating element so that the end portions of the virus removal bag overlap each other. A plurality of virus removal bags can be simultaneously attached to the rotating element, and this method is advantageous for treating a large volume of a virus-containing suspension at once.

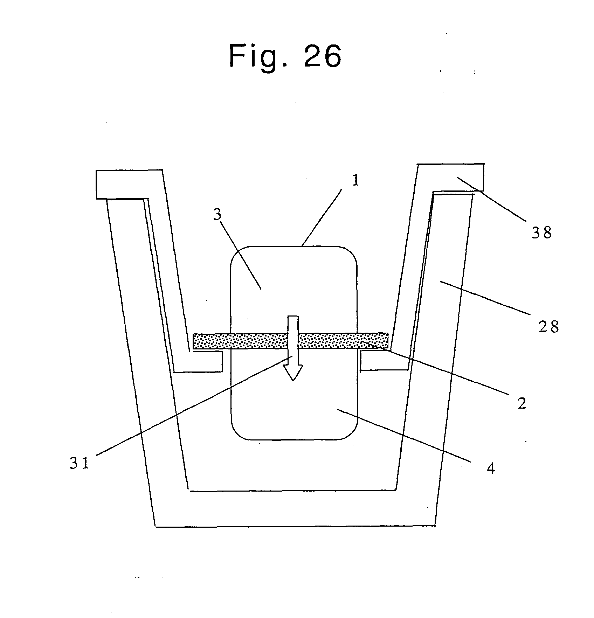

Another method for promoting the filtration by applying a centrifugal force to a virus removal bag having a separation membrane (b) in the form of a sheet is shown in Fig. 26. Fig. 26 shows a schematic view explaining an example of a virus removal bag placed in a centrifuge cup, wherein the virus removal bag has the same structure as that of the virus removal bag shown in Fig. 1. In Fig. 26, centrifuge cup 28 is provided with auxiliary pot 38 for receiving a virus removal bag therein, and the virus removal bag is securely held in auxiliary pot 38 on the fixation collar of the bag which is provided for fixing the separation membrane (b) in the pouchy casing (a) (see Fig. 8(b)). When the virus removal bag is attached to the centrifuge cup in such a manner, it becomes possible to suppress the movement of the whole of the virus removal bag in the centrifugal direction. The rotation of the rotating element of a centrifuge, which has a virus removal bag attached thereto in the above-mentioned manner, causes a centrifugal force to be exerted in direction 31 indicated by a white arrow in Fig. 26, and this promotes the filtration.

The application of a pressure to the virus -containing suspension in the first compartment (c) is performed so that the virus-containing suspension in the first compartment (c) is caused to move through the virus removal membrane into the second compartment (d), thereby forming a filtrate. The use of a pressure for promoting the filtration is especially suitable when using a virus removal bag containing a filter bag. The amount of a pressure applied to the first compartment (c) may be selected based on the pressure resistance of the separation membrane (b) or the filter bag. When the virus removal bag of the present invention is used for the filtration, it is preferred that the pressure applied to the membrane surface is in the range of from 0.05 to 100 kg/cm$^2$. As examples of methods for applying a pressure to a virus-containing suspension, there can be mentioned a method in which a virus removal bag is directly pressurized using a roll-type pressurizing apparatus or a plate-type pressurizing apparatus, to thereby collect a virus-removed suspension as a filtrate in a second compartment (d); and a method in which a virus-containing suspension is introduced into a filter bag to which a bag containing gas for pressurization is connected, and the inside of the filter bag is pressurized by applying a pressure to the bag containing gas for pressurization, to thereby filter the virus-containing suspension.

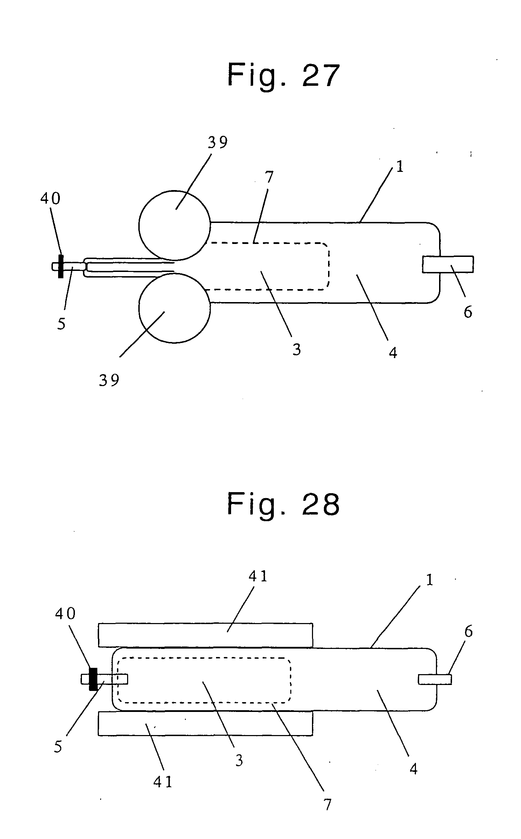

Fig. 27 shows a schematic view explaining an example of a method for performing the filtration by using a roll-type pressurizing apparatus for applying a pressure to a virus removal bag. In the apparatus shown in Fig. 27, roll 39 of a roll-type pressurization apparatus rotates slowly to compress filter bag 7 contained in a virus removal bag having pouchy casing 1, thereby applying a pressure to the virus removal bag. As a result, a filtrate obtained by filtering the virus -containing suspension is gradually collected in a second compartment (d). Pinchcock 40 provided at an outer terminal portion of a tube used as inlet 5 prevents the backflow of the virus-containing suspension (i.e., flow of the virus-containing suspension in a direction opposite to the direction of the filtration). Instead of using a pinchcock, the terminal portion of the tube can be sealed by melt-sealing.

Fig. 28 shows a schematic view explaining an example of a method for performing the filtration by using a plate-type pressurizing apparatus for applying a pressure to a virus removal bag. In the apparatus shown in Fig. 28, filter bag 7 is positioned between two plates 41 of the plate-type pressurization apparatus, and two plates 41 gradually decrease the distance therebetween, to thereby apply a pressure to virus removal bag 7. As a result, a filtrate obtained by filtering the virus-containing suspension is gradually collected in the second compartment (d).

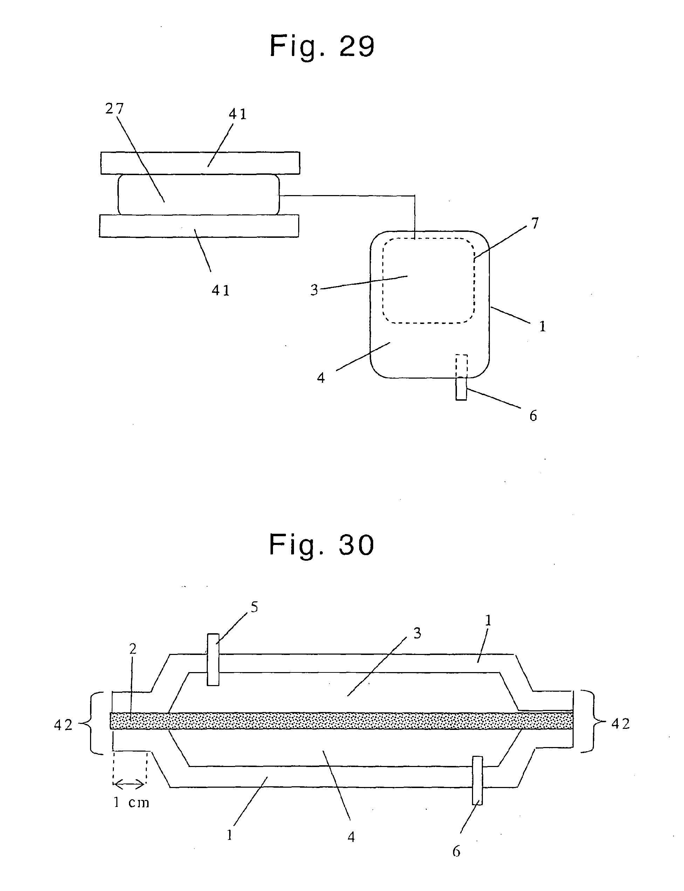

Fig. 29 shows a schematic view explaining an example of a method for pressurizing a virus removal bag by using a bag containing a gas for pressurization. In the apparatus shown in Fig. 29, a gas having low reactivity, such as air, a nitrogen gas or an argon gas, is enclosed in bag 27 which is a bag containing gas for pressurization. Bag 27 is pressurized using plates 41 of a plate-type pressurizing apparatus to introduce into filter bag 7 the gas contained in bag 27, thereby pressurizing the virus removal bag. As a result, a filtrate obtained by filtering the virus-containing suspension is gradually collected in the second compartment (d).

In any of the above-mentioned methods, it is preferred that the pressurization of the virus removal bag is performed at a temperature in the range of from 0 to 40 °C, more preferably from 5 to 35 °C. When the pressurization is performed at a temperature in the above -mentioned range, even when the virus-containing suspension is plasma, the filtration through the virus removal membrane can be performed at a high speed without causing serious denaturation of the virus -containing suspension.

Since a filtrate (which is a virus-removed suspension) is collected in the second compartment (d), in step (5), the virus-removed suspension is withdrawn through the outlet from the second compartment (d). The virus-removed suspension may be withdrawn by opening the outlet.

There is no particular limitation with respect to the virus-containing suspension used in the virus removal method of the present invention, and there can be mentioned a culture broth containing viruses, and body fluids, such as blood and plasma, which are suspected to contain viruses. It is preferred that the virus -containing suspension used in the present invention is blood or plasma of human or animal. In the present invention, the term "plasma" means a blood component which is obtained by removing blood cell components, such as erythrocytes, leukocytes and platelets, from whole blood. For example, the plasma used in the present invention is a supernatant obtained by centrifuging collected whole blood or plasma component obtained using an apheresis instrument. However, in the present invention, plasma used as a virus-containing suspension may have a blood cell content of not more than 10 % by weight.

It is preferred that the plasma used in the present invention has never been frozen. In general, plasma prepared as a blood product is frozen within several hours from blood collection, but when the frozen plasma is thawed and subjected to filtration, the filtration properties of the plasma are changed and such plasma is likely to cause clogging of the filter. Herein, the term "freezing" means a treatment in which plasma is maintained at a low temperature to thereby change the plasma from a liquid state to a solid state, or plasma is caused to experience such thermal history at least once. When plasma which has never been frozen is used in the virus removal method of the present invention, it becomes possible to obtain a virus-removed plasma in a practically useful amount within a short period of time.

Further, it is preferred that the plasma used in the present invention is leukocyte-removed plasma. Leukocyte-removed plasma is plasma which has been subjected to a leukocyte removal process and in which the leukocyte concentration has been lowered to not more than 1/10, preferably not more than 1/100 of the leukocyte concentration of the plasma before subjected to the leukocyte removal process. Since filtration interrupting substances, such as leukocytes and impurities remaining in plasma, are removed by the leukocyte removal process, a stable filtration becomes easy by the use of the leukocyte-removed plasma. There is no particular limitation with respect to the leukocyte removal method for preparing the leukocyte-removed plasma. Whole blood, plasma or a plasma-containing component can be filtered through a leukocyte removal filter, to thereby obtain leukocyte-removed plasma. As examples of methods for directly subjecting whole blood obtained by blood collection to a leukocyte removal process, there can be mentioned a method in which collected whole blood is centrifuged to thereby separate plasma component from whole blood, and the separated plasma is subjected to a leukocyte removal process; or a method in which plasma component obtained using an apheresis instrument is subjected to a leukocyte removal process. When the removal of filtration interrupting substances by the above-mentioned leukocyte removal method is unsatisfactory, thus rendering difficult the subsequent virus removal operation, an adsorption treatment using an adsorbent or a membrane filtration by a microfilter may be performed prior to the virus removal method of the present invention, to thereby remove the remaining filtration interrupting substances.

In the present invention, it is preferred that the second compartment (d) of the virus removal bag used for removing viruses has a volume sufficient to collect all of the filtrate obtained by filtering a virus-containing suspension through the virus removal membrane. Accordingly, in the method of the present invention for removing viruses, it is preferred that, in step (4), all of the filtrate obtained by filtering the virus-containing suspension through the virus removal membrane is collected in the second compartment (d) before performing step (5). When all of the filtrate obtained by filtering a virus-containing suspension is collected in the second compartment (d), there is no danger of incomplete filtration caused by backflow of the filtrate into the first compartment (c). Further, in the method of the present invention, there is no need to recover the filtrate (i.e., a virus-removed suspension) from the virus removal bag before completing the filtration operation or to provide an additional bag for collecting the filtrate, wherein the additional bag is separately connected through a liquid transfer pipe and the like to the virus removal bag. Accordingly, a virus-removed suspension can be obtained by easy operations.

In another embodiment of the present invention, there is provided a method for preparing a virus-removed plasma, comprising:

(1) providing a closed, virus removal system which comprises:

a plasma collector means (i) for collecting whole blood comprising plasma and blood cells and separating and collecting plasma from whole blood, wherein the whole blood is suspected to contain viruses, at least one virus removal bag (ii), and

a plasma recovery means (iii) for recovering a virus-removed plasma,
the plasma collector means (i) being aseptically and fluid-tightly connected to the virus removal bag (ii), and
the virus removal bag (ii) being aseptically and fluid-tightly connected to the plasma recovery means (iii);

(2) collecting whole blood from a donor into the plasma collector means (i);
(3) separating the collected whole blood into plasma and blood cells by centrifugation;
(4) introducing the separated plasma into the at least one virus removal bag (ii) from the plasma collector means (i), to receive the separated plasma in the first compartment (c) of the virus removal bag (ii);
(5) filtering the separated plasma through the virus removal membrane of the virus removal bag (ii), to thereby remove viruses from the separated plasma;
(6) collecting a filtrate which is a virus -removed plasma in the second compartment (d) of the virus removal bag (ii); and
(7) discharging the virus-removed plasma from the virus removal bag (ii) into the plasma recovery means (iii).

In the method of the present invention for preparing a virus-removed plasma, as a first step, there is provided a closed, virus removal system which comprises a plasma collector means (i) for collecting whole blood comprising plasma and blood cells and separating and collecting plasma from whole blood, wherein the whole blood is suspected to contain viruses; at least one virus removal bag (ii); and a plasma recovery means (iii) for recovering a virus-removed plasma, wherein the plasma collector means (i) is aseptically and fluid -tightly connected to the virus removal bag (ii), and the virus removal bag (ii) is aseptically and fluid -tightly connected to the plasma recovery means (iii). The virus removal system used in the present invention is a system obtained by adding the virus removal bag of the present invention to a conventional multi-bag system for collecting blood components, wherein the conventional multi-bag system has been used for collecting plasma. As such a virus removal system, there can be used the above-mentioned multi-bag virus removal system for obtaining a virus-removed plasma. Specific examples of virus removal systems include systems which are schematically shown in Figs. 13 to 22. In these drawings, the functional bags and the like which are located upstream of the virus removal bag constitute the plasma collector means (i), and the functional bags and the like which are located downstream of the virus removal bag constitute the plasma recovery means (iii). Since even when used alone the virus removal bag of the present invention is capable of removing viruses from plasma, the method of the present invention is advantageous in that it enables preparation of a virus-removed plasma without largely altering the conventional process for producing blood products and transfusable plasma from donated blood.

In step (2) of the method of the present invention, whole blood from a donor is collected into the plasma collector means (i) of the virus removal system, and in step (3), the collected whole blood is separated into plasma and blood cells by centrifugation. The collection of whole blood and separation of plasma may be performed by any conventional method.

Next, in steps (4) to (6), viruses are removed from separated plasma by using the virus removal bag (ii). The virus removal may be performed by the above -mentioned method of the present invention for removing viruses from a virus-containing suspension. Specifically, the virus removal is performed by a method comprising:

introducing the separated plasma into the at least one virus removal bag (ii) from the plasma collector means (i), to receive the separated plasma in the first compartment (c) of the virus removal bag (ii);
filtering the separated plasma through the virus removal membrane of the virus removal bag (ii), to thereby remove viruses from the separated plasma; and
collecting a filtrate which is a virus-removed plasma in the second compartment (d) of the virus removal bag (ii).

For preventing the denaturation of plasma, it is preferred that the virus removal process is performed as quickly as possible after collecting blood by blood donation and the like. Specifically, when the temperature is in the range of from 20 to 40 °C, it is preferred that the virus removal of plasma is performed within 10 hours, more advantageously within 6 hours from blood collection. When the temperature is in the range of from 10 to 20 °C, it is preferred that the virus removal of plasma is performed within 24 hours from blood collection.

Finally, in step (7), the virus-removed plasma is discharged from the virus removal bag (ii) into the plasma recovery means (iii). The collected plasma can be immediately used for producing plasma products or for plasma transfusion. Alternatively, the collected plasma can be stored under freezing.

In the present invention, it is preferred that the second compartment (d) of the virus removal bag contained in the virus removal system has a volume sufficient to collect all of the filtrate obtained by filtering plasma through the virus removal membrane. Accordingly, in the method of the present invention, it is preferred that, in step (6), all of the filtrate obtained by filtering the separated plasma is collected in the second compartment (d) of the virus removal bag (ii) before performing step (7). When all of the filtrate obtained by filtering plasma is collected in the second compartment (d), there is no danger of incomplete filtration caused by the backflow of the filtrate into the first compartment (c).

Further, in the method of the present invention, there is no need to recover the filtrate (i.e., a virus-removed plasma) from the virus removal bag before completing the filtration operation or to provide an additional bag for collecting the filtrate, wherein the additional bag is connected through a liquid transfer pipe and the like to the virus removal bag. Therefore, a virus-removed plasma can be obtained by easy operations while lowering the possibility of breaking the aseptic conditions.

In still another embodiment of the present invention, there is provided human or animal plasma obtained by the above-mentioned method for preparing a virus -removed plasma. By the use of the method of the present invention, all viruses, including viruses in the window period and viruses which are not covered by the virus detection methods employed in the field of blood treatment, are removed from plasma to thereby provide a virus-removed plasma, and such a virus-removed plasma is advantageous as a raw material for plasma products and as transfusable plasma.

BEST MODE FOR CARRYING OUT THE INVENTION

Hereinbelow, the present invention will be described in more detail with reference to the following Examples and Reference Examples, which should not be construed as limiting the scope of the present invention.

In the following Examples and Reference Examples, various properties of each of filters and virus removal bags were measured and evaluated as follows.

[The average pore diameter of a filter]

The average pore diameter of a filter was measured in accordance with the methods prescribed under ASTM F316-86 and E128-61. Specifically, the measurement of the average pore diameter of a filter was performed as follows. A stamped-out circular filter having a diameter of 25 mm was set in a cell. Then, a perfluorocarbon coolant (FX 3250; manufactured and sold by Sumitomo 3M Ltd., Japan), which was used as a filler liquid, was filled into the cell. Using air, a pressure was applied to one side of the filter in the cell while gradually increasing the pressure. When the flow rate of the air permeating through the filter reached 2.5 ml per minute as measured by a flow meter located on the other side of the filter (remote from the side to which the air pressure was applied), the value of the applied pressure P (Pa) was read. Using the thus obtained value P, the average pore diameter of the filter was calculated by the following formula (1):

$$\text{Average pore diameter } (\mu m) = 34{,}320/P \tag{1}$$

[The log reduction value (LRV) of bovine viral diarrhea virus]

MDBK cells cultured in Dulbecco's MEM (manufactured and sold by Nikken Biomedical Laboratories, Japan) containing 5 % equine serum were infected with bovine viral diarrhea virus, to thereby form a culture supernatant of virus-infected cells, and the culture supernatant was collected to thereby obtain a virus -containing suspension. A part of the obtained virus -containing suspension was filtered through a virus removal membrane under conditions wherein the pressure was 0.1 MPa and the temperature was 25 °C, and the resultant filtrate was collected in the form of 10 fractions each containing 2 ml of the filtrate. 1 ml of a sample was obtained from each of the 10 fractions and mixed together, thereby obtaining a virus-removed suspension. The virus concentration of each of the virus -containing suspension (before filtration) and the virus-removed suspension (which is a filtrate) was determined by the $TCID_{50}$ method using cultured MDBK cells, and the log reduction value was calculated based on the determined virus concentrations of the virus-containing suspension and the virus-removed suspension. The log reduction value (LRV) is represented by the following formula (2):

$$\text{the log reduction value (LRV)} = -\log_{10} (\text{virus}$$

$$\text{concentration of a filtrate which is a virus}$$

$$\text{-removed suspension})/(\text{virus concentration of a}$$

$$\text{virus-containing suspension before filtration}) \tag{2}$$

[The volume of a second compartment (d)]

The volume of a second compartment (d) of a virus removal bag was determined as follows. The point of contact between an outlet and a second compartment of a virus removal bag was pinched using a pinchcock and pure water was added to the second compartment. The resultant virus removal bag containing pure water was caused to stand still in the upright position or alternatively, the resultant virus removal bag was hung down in the upright position, followed by measuring the volume of pure water contained in the portion of the second compartment (d) which corresponds to the hatched portion shown in Fig. 7. The measured value was used as the volume of the second compartment (d).

[The perviousness for latex particles]

100 ml of a 1 % by weight aqueous suspension of a styrene latex (having an average particle diameter of 120 nm) (manufactured and sold by Asahi Kasei Corporation, Japan; hereinafter referred to as "latex suspension") was introduced to a blood bag for whole blood in a virus removal system. The whole of the latex suspension was introduced to a first compartment (c) of the virus removal bag in the virus removal system. Then, the virus removal bag containing the latex suspension was centrifuged using a centrifuge (Model 8100; manufactured and sold by Kubota Corporation, Japan) at 25 °C under 1000 G for 15 minutes, to thereby obtain a filtrate in a second compartment (d) of the virus removal bag. The particle size distribution of the latex particles contained in the filtrate was determined using a particle size analyzer "Particle Size Analyzer UPA150, Model 9230" (manufactured and sold by Microtrac Inc., U.S.A.).

Example 1

A mixture of 40 % by weight of polyvinylidene fluoride resin (SOLEP1012; manufactured and sold by Solvay Solexis K.K., Japan; the crystalline melting point: 173 °C), and 60 % by weight of dicyclohexyl phthalate (for industrial use; manufactured and sold by Osaka Organic Chemical Industry LTD., Japan) was subjected to melt-kneading at 200 °C using a kneader (Labo Plastomill Model C; manufactured and sold by Toyo Seiki Seisaku-sho, Ltd., Japan). The resultant molten mixture was cooled to a temperature of 30 °C or less, thereby obtaining a bulk of resin. The bulk of resin was subjected to hot-press at 200 °C under a pressure of 10 MPa, followed by cold-press under a pressure of 10 MPa, thereby obtaining a resin sheet. Subsequently, the dicyclohexyl phthalate contained in the resin sheet was removed by extraction using, as an extractant, isopropyl alcohol (special grade reagent) (manufactured and sold by Wako Pure Chemical Industries, Ltd., Japan), to thereby obtain a porous membrane A. The porous membrane A had an average pore diameter of 83 nm and a thickness of 40 $\mu$m.

On the other hand, a porous membrane was prepared in substantially the same manner as in the preparation of porous membrane A described above, except that the amount of polyvinylidene fluoride resin was changed to 25 % by weight, and that 75 % by weight of diethylhexyl phthalate was used instead of 60 % by weight of dicyclohexyl phthalate, thereby obtaining a porous membrane B. The porous membrane B obtained had an average pore diameter of 123 nm and a thickness of 42 $\mu$m.

With respect to each of the porous membranes A and B, a treatment for imparting hydrophilicity was performed as follows. First, each of the porous membranes A and B was irradiated with $Co^{60}$ $\gamma$-ray at a dose of 100 kGy in an atmosphere of nitrogen gas. On the other hand, hydroxyethyl methacrylate (first grade reagent) (manufactured and sold by Wako Pure Chemical Industries, Ltd., Japan) and polyethylene glycol diacrylate (manufactured and sold by Sigma-Aldrich Corporation, U.S.A.) were dissolved in 1-butanol so as to obtain a hydrophilic compound solution in which the concentrations of hydroxyethyl methacrylate and polyethylene glycol diacrylate were 10 % by weight and 1 % by weight, respectively. Each of the porous membranes A and B after irradiation of $\gamma$-ray was individually immersed in the above-obtained hydrophilic compound solution at 40 °C for 2 hours, thereby effecting a reaction. From each of the resultant reaction mixtures, the membrane was recovered and washed with ethanol, followed by drying, thus obtaining dried membranes A and B. Further, each of the dried membranes was immersed in hot water using an autoclave at 121 °C for 20 minutes, thereby obtaining hydrophilic porous membranes A and B.

The hydrophilic porous membranes B and A obtained above were laminated on each other in this order as viewed in a flow direction of the virus-containing suspension, thereby obtaining a composite membrane. With respect to the composite membrane, the log reduction value for bovine viral diarrhea virus was measured, and it was found that the log reduction value was 3.1.

The thus obtained composite membrane was sandwiched between two nonwoven polyester fabrics (each having a weight per unit area of 50 g/m$^2$) (manufactured and sold by Asahi Kasei Corporation, Japan), to thereby obtain a composite filter, and the obtained composite filter was cut into a square of a 20 cm $\times$ 20 cm size. The peripheral portions of the composite filter (which correspond to the portion indicated by reference numeral 11 in Fig. 5) were subjected to heat-sealing so as to form a heat-sealed peripheral portion having a width of about 2 mm. In the obtained composite filter, the porous membrane B was used as a prefilter and the porous membrane A was used as a virus removal filter.

Two composite filters each having a 11 cm × 11 cm size were cut out from the above-obtained composite filter. The two composite filters were placed on top of each other so that the porous membranes B of the two composite filters were faced with each other, to thereby form a laminate. The laminate was heat-sealed as indicated in Fig. 6(a), thereby obtaining a filter bag having a 11 cm × 11 cm size. The width of the heat -sealed portion of the filter bag was about 4 mm. Then, the filter bag was put in a flexible, soft polyvinyl chloride (PVC) pouchy casing (a) having a size of 13 cm × 20 cm and having a tube for use as an outlet. Further, a PVC tube was partly inserted into the filter bag so as to form an inlet. The filter bag having the PVC tube partly inserted therein as an inlet, was placed in the flexible PVC pouchy casing (a) so that the outer end of the PVC tube as an inlet was positioned outside of the pouchy casing (a), and the pouchy casing (a) was heat-sealed as shown in Fig. 9(b), thereby obtaining a virus removal bag having a separation membrane (b) in the form of a bag (i.e., a filter bag). In the thus obtained virus removal bag, a membranous, overall surrounding wall of the filter bag partitions the internal space of the pouchy casing (a) into a first compartment (c) which is the internal space of the filter bag communicating with the inlet and a second compartment (d) which is the internal space of the pouchy casing (a), exclusive of a filter bag portion, communicating with the outlet. The volume of the second compartment (d) of the virus removal bag was measured, and it was found that the second compartment (d) had a volume of 180 $cm^3$.

A first tube was connected to the inlet of the filter bag, and the virus removal bag having the filter bag was connected to a blood bag for whole blood (volume: 200 ml) through the first tube. Further, a second tube was connected to the outlet of the pouchy casing, and the virus removal bag (having the pouchy casing) was connected to a blood bag for filtered plasma (volume: 200 ml) through the second tube, thereby obtaining a multi-bag virus removal system as schematically shown in Fig. 13. In the production of the multi-bag virus removal system, the second compartment (d) of the virus removal bag was degassed before connecting the virus removal bag to the blood bags. The multi-bag virus removal system was subjected to autoclaving sterilization at 121 °C for 20 minutes, thereby obtaining a closed, multi-bag virus removal system.

Using the thus obtained, multi-bag virus removal system as shown in Fig. 13, a virus-removed plasma was prepared as follows.

First, 200 ml of whole blood was taken from a donor and collected in blood bag 20. Then, the whole system in which the virus removal bag was connected to the blood bags, was loaded into a centrifuge cup of a centrifuge "himac CR7 Model B3" (manufactured and sold by Hitachi High-Technologies Corporation, Japan). A centrifugation of the whole blood in blood bag 20 was performed under 4000 G for 10 minutes, thereby separating the whole blood into a supernatant phase comprised of a plasma component and a precipitate phase comprised of a blood cell component. At the point in time of completion of the centrifugation, no damage was found in any of the virus removal bag and the blood bags connected thereto. The plasma component obtained above in a volume of about 100 ml was carefully introduced into the first compartment (c) (i.e., the filter bag) of the virus removal bag so as not to allow entry of the blood cell component into the first compartment (c) (filter bag). After completion of the introduction of the plasma component into the first compartment (c), the first tube (connecting blood bag 20 for whole blood to the inlet of the virus removal bag having pouchy casing 1) was cut off by melt-seal/cutting at portion 19 of liquid transfer pipe. Consequently, the multi-bag virus removal system was comprised of the virus removal bag (having pouchy casing 1) and the blood bag 21 for filtered plasma. Then, the second tube connected to the outlet of the virus removal bag (having pouchy casing 1), wherein the outlet communicated with the second compartment (d) of the virus removal bag, was pinched using a pinchcock, so that the whole of a filtered plasma was able to be collected and held in the second compartment (d) of the virus removal bag. Subsequently, as indicated in Fig. 23, the virus removal bag was loaded into a modified centrifuge cup of a centrifuge. Specifically, in the modified centrifuge cup, securing bar 30 for the virus removal bag was provided across the inside of the centrifuge cup, and a securing hook indicated in Fig. 24 (i.e., securing hook 29 indicated in Fig. 23) was provided at the top end of the virus removal bag placed in the centrifuge cup, and the securing hook 29 of the virus removal bag in the centrifuge cup was securely engaged with the securing bar 30. In the modified centrifuge cup, blood bag 21 for filtered plasma was positioned on the bottom of the cup. The modified centrifuge cup containing the virus removal bag was loaded onto a centrifuge (Model 8100; manufactured and sold by Kubota Corporation, Japan), and a centrifugation was performed at 25 °C under 1000 G for 30 minutes. As a result, the plasma component in the filter bag was filtered therethrough, and all of the resultant filtered plasma was collected in the second compartment (d) of the virus removal bag. The weight of the filtered plasma was measured. Using the obtained value of the weight of the filtered plasma, the yield of the filtered plasma was calculated by the formula below. As a result, it was found that the yield was 95 %.

Yield (%) = [(the weight (g) of the filtered

plasma collected in the second compartment

(d))/(the weight (g) of the plasma introduced

into the first compartment (c))] $\times$ 100

At the point in time of completion of the centrifugation, no damage was found in any of the virus removal bag and the blood bags connected thereto.

As apparent from the above, the method of the present invention for preparing a virus-removed plasma is advantageous in that a desired volume of virus-removed plasma can be aseptically obtained by a series of consecutive operations, without using a sterile room and without the problem of clogging of a membrane.

Further, substantially the same multi-bag virus removal system as described above was subjected to measurement of the perviousness for latex particles. As a result, it was found that with respect to the virus removal bag of the above-mentioned multi-bag virus removal system, the perviousness for latex particles was below the detection limit. Therefore, it was confirmed that the permeation of particles having an average pore diameter of 120 nm, such as viruses, into the filtered plasma, is prevented by the virus removal bag of the present invention.

Example 2

A virus removal bag for use in a closed, multi-bag virus removal system was produced in the same manner as in Example 1. Using the obtained virus removal bag, a closed, multi-bag virus removal system as indicated in Fig. 21 was produced. The system produced herein contained a leukocyte removal unit 22. As a leukocyte removal unit 22, a commercially available product "Sepacell" (trade name; manufactured and sold by Asahi Medical Corporation, Japan) was used.

A plasma component was separated from whole blood in substantially the same manner as in Example 1. The separated plasma component was filtered through the above-mentioned leukocyte removal unit 22, thereby obtaining leukocyte-removed plasma. The leukocyte -removed plasma was introduced into a first compartment (c) (i.e., a filter bag) of the virus removal bag (having pouchy casing 1), and then virus-removal was performed in substantially the same manner as in Example 1. With respect to the resultant virus-removed plasma (i.e., filtered plasma), it was found that the yield of the filtered plasma was 97 %. That is, the yield of the filtered plasma achieved in Example 2 was even higher than in Example 1 in which a leukocyte removal step was not performed. At the point in time of completion of the centrifugation, no damage was found in any of the virus removal bag and the blood bags connected thereto.

In the case of the closed, multi-bag virus removal system produced in this Example 2, when whole blood was centrifuged for separation thereof into a blood cell component and a plasma component, no damage was caused to any of the virus removal bag and the blood bags connected thereto, in spite of the presence of the leukocyte removal unit in the system.

Example 3

A hydrophilic porous membrane was produced in substantially the same manner as in Example 1, except:

that a polyethylene porous membrane (having an average pore diameter of 65 nm and a thickness of 20 $\mu$m) (manufactured and sold by Asahi Kasei Corporation, Japan) was used as a porous membrane (which was subjected to hydrophilicity-imparting treatment by addition -bonding of a hydrophilic compound);
that a hydrophilic compound solution was prepared by dissolving hydroxyethyl methacrylate (first grade reagent; manufactured and sold by Wako Pure Chemical Industries, Ltd., Japan) in methanol so that the final concentration of hydroxyethyl methacrylate became 50 % by weight; and
that the reaction was performed at 40 °C for 15 minutes.

The hydrophilic porous membrane obtained had an average pore diameter of 58 nm.

Subsequently, a composite filter was produced in substantially the same manner as in Example 1, except that, instead of porous membrane A, the above-obtained hydrophilic porous membrane was used as a virus removal filter. With respect to the composite filter obtained, the log reduction value for bovine viral diarrhea virus was measured, and it was found that the log reduction value was 3.9.

Using the above-obtained composite filter, a filter bag was produced in the same manner as in Example 1. Then, a virus removal bag was produced in substantially the same manner as in Example 1 except that the above -obtained filter bag was used. Using the thus produced virus removal bag, a multi-bag virus removal system was produced in the same manner as in Example 1.

Using the thus produced multi-bag virus removal system, virus-removed plasma was prepared in substantially the same manner as in Example 1. With respect to the virus-removed plasma prepared (i.e., filtered plasma), it was found that the yield of the filtered plasma was 92 %. Further, with respect to the virus removal bag, it was found that the perviousness for latex particles was below the detection limit.

The results above show that a virus removal filter (comprising a hydrophilic porous membrane) obtained by imparting hydrophilicity to a polyethylene porous membrane is also usable for the production of the virus removal bag of the present invention.

Example 4

A virus removal filter (comprising a hydrophilic porous membrane) was produced in substantially the same manner as in Example 3, except that, instead of hydroxyethyl methacrylate, hydroxypropyl acrylate was used as a hydrophilic compound. A composite filter was produced using such a virus removal filter, which had an average pore diameter of 52 nm, and the composite filter exhibited a log reduction value (LRV) of 3.9 for bovine viral diarrhea virus.

Using the above-produced composite filter, a filter bag was produced in the same manner as in Example 1. Then, a virus removal bag was produced in substantially the same manner as in Example 1 except that the above - produced filter bag was used. Using the thus produced virus removal bag, a multi-bag virus removal system was produced in the same manner as in Example 1.

Using the thus produced multi-bag virus removal system, virus-removed plasma was prepared in substantially the same manner as in Example 1. With respect to the virus-removed plasma prepared (i.e., filtered plasma), it was found that the yield of the filtered plasma was 94 %. Further, with respect to the virus removal bag, it was found that the perviousness for latex particles was below the detection limit.

The results above show that a virus removal filter (comprising a hydrophilic porous membrane) obtained by imparting hydrophilicity to a polyethylene porous membrane by using hydroxypropyl acrylate is also usable for the production of the virus removal bag of the present invention.

Example 5

A virus removal bag was produced in substantially the same manner as in Example 1, except that a spongy adsorber (a melamine foam having a size of 80 mm × 80 mm × 10 mm) (manufactured and sold by Arai-Kasei Co., Ltd., Japan) was placed inside the filter bag, thereby obtaining a virus removal bag having a filter bag (i.e., a first compartment (c)) containing a spongy adsorber.

A multi-bag virus removal system was produced in substantially the same manner as in Example 1, except that the above-produced virus removal bag was used and a bag for removing air from the spongy adsorber (i.e., bag 26 for removing air from the virus removal bag) was connected to the filter bag, thereby obtaining a multi -bag virus removal system as indicated in Fig. 22. Bag 26 for removing air from the virus removal bag was an empty bag containing no air. The air contained in the filter bag was transferred into bag 26 during the introduction of plasma into the first compartment (c) (i.e., the filter bag) of the virus removal bag. Specifically, plasma component was separated from whole blood in the same manner as in Example 1, and the separated plasma component was introduced into the filter bag containing the spongy adsorber while transferring the air contained in the spongy adsorber into bag 26 for removing air from the virus removal bag. On the other hand, the air contained in a second compartment (d) of the virus removal bag had been emptied. Each of a tube for introducing the plasma component into the filter bag and another tube connecting the filter bag to bag 26 for removing air from the virus removal bag was cut off by melt-seal/cutting at portion 19 indicated in Fig. 22. Then, a part of the tube connected to the outlet of the virus removal bag, which outlet communicates with the second compartment (d) of the virus removal bag, was pinched using a pinchcock, so that all of filtered plasma was able to be collected and held in the second compartment (d) of the virus removal bag. Subsequently, using a roll-type pressurization apparatus, the plasma component was gradually filtered through the filter bag over 30 minutes, thereby collecting a filtered plasma in the second compartment (d). A schematic view explaining the filtration performed using a roll-type pressurizing apparatus is shown in Fig. 27. As a result, the plasma component in the filter bag was filtered therethrough, and all of the resultant filtered plasma (which is a virus-removed plasma) was collected in the second compartment (d) of the virus removal bag.

With respect to the filtered plasma, it was found that the yield of the filtered plasma was 92 %. Further, with respect to the virus removal bag, it was found that the perviousness for latex particles was below the detection limit.

The results above show that, in the present invention, filtration of a virus-containing suspension can be promoted by applying a pressure to the virus -containing suspension in the first compartment (c) by using a roll-type pressurization apparatus.

Example 6

Virus-removed plasma was prepared in substantially the same manner as in Example 5, except that a plate -type pressurization apparatus was used instead of a roll-type pressurization apparatus. Specifically, using a plate-type pressurization apparatus, filtration of the plasma component was performed gradually over 30 minutes, thereby collecting a filtered plasma in the second compartment (d). A schematic view explaining the filtration performed using a plate-type pressurization apparatus is shown in Fig. 28. Thus, the plasma component in the filter bag was filtered therethrough, and all of the resultant filtered plasma (which is a virus-removed plasma) was collected in the second compartment (d) of the virus removal bag.

With respect to the filtered plasma, it was found that the yield of the filtered plasma was 94 %. Further, with respect to the virus removal bag, it was found that the perviousness for latex particles was below the detection limit.

The results above show that, in the present invention, filtration of a virus-containing suspension can be promoted by applying a pressure to the virus -containing suspension in the first compartment (c) by using a plate-type pressurization apparatus.

Example 7

Two casing halves (first and second casing halves) each having the shape of a bowl (height: 1.5 cm, length: 20 cm, width: 10 cm) were prepared from a flexible, soft polyvinyl chloride sheet having a thickness of 0.4 mm. The casing halves were each prepared so as to have a fixation collar (width: 1 cm) at the periphery thereof. Using an epoxy resin adhesive, a tube (for use as an inlet) was attached to the first casing half and another tube (for use as an outlet) was attached to the second casing half. The composite filter produced in Example 1 was provided for use as a separation membrane (b). The separation membrane (b) was sandwiched between the two casing halves so as to form a structure in which the bottom walls of the casing halves are positioned remote from each other and the fixation collars of the casing halves are positioned to face each other through the peripheral portion of the separation membrane (b). The fixation collars of the first and second casing halves were adhered to each other through the peripheral portion of the separation membrane (b) by heat sealing, to thereby obtain a virus removal bag. The cross-sectional view of the virus removal bag obtained above is shown in Fig. 30. In Fig. 30, the fixation collar is indicated by reference numeral 42, the first casing half used as a first compartment (c) is indicated by reference numeral 3, and the second casing half used as a second compartment (d) is indicated by reference numeral 4.

The above-prepared virus removal bag was laid onto the outer side surface of a cylindrical rotor (i.e., rotating element) having a diameter of 20 cm and, then, the virus removal bag was screwed to the rotor, thereby securing the former to the latter. A schematic view of the resultant rotating element having a virus removal bag attached thereto is shown in Fig. 25(b). About 100 ml of the plasma component used in Example 1 was introduced into a first compartment (c) of the virus removal bag, and a centrifugation was performed at 25 °C under 1000 G for 30 minutes. As a result, the plasma component in the first compartment (c) was filtered through the separation membrane (b) which is a composite filter, and all of the resultant filtered plasma was collected in the second compartment (d) of the virus removal bag. The yield of the filtered plasma was 90 %.

The results above show that virus-removed plasma can be obtained in high yield by using the virus removal bag having a separation membrane (b) in the form of a sheet.

Reference Example 1

A filter bag was produced in substantially the same manner as in Example 1, except that a nonwoven polyester fabric (manufactured and sold by Asahi Kasei Corporation, Japan) alone (i.e., without using a virus removal filter and a prefilter) was used instead of a composite filter. Using the produced filter bag, a virus removal bag was produced in the same manner as in Example 1. The nonwoven fabric used herein corresponds to the leukocyte removal filter disclosed in Unexamined Patent Application Laid-Open Specification No. Hei 7-267871.

The virus removal bag obtained above was subjected to measurement of the perviousness for latex particles. As a result, it was found that 78 % by volume of the latex particles contained in the latex suspension introduced into the first compartment (c) of the virus removal bag moved through the filter bag into the second compartment (d) of the virus removal bag. Therefore, a filter made of a nonwoven fabric was incapable of preventing particles having an average particle diameter of 120 nm from passing therethrough.

Reference Example 2

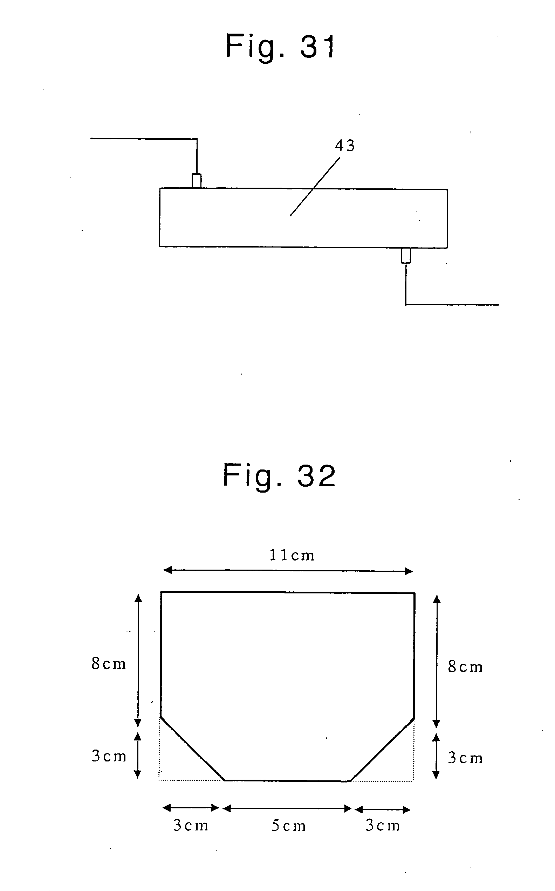

A multi-bag virus removal system was produced in substantially the same manner as in Example 1, except that a casing shown in Fig. 31 (i.e., a cylindrical, hard polysulfone casing having a diameter of 3 cm and a length of 13 cm) was used instead of the virus removal bag. The above-mentioned casing was used as a dummy of a conventional hollow fiber filter module.

Whole blood was introduced into a blood bag for whole blood of the produced multi-bag virus removal system, and all of the multi-bag virus removal system was subjected to centrifugation under the same conditions as in Example 1. Although no serious damage, such as a tear, was caused to the blood bags contained in the multi-bag virus removal system, disadvantageous phenomena, such as formation of scratches on the surfaces of the blood bags, were caused by the collision of the cylindrical casing with the blood bags.

Example 8

A multi-bag virus removal system was produced in substantially the same manner as in Example 1, except that the filter bag employed was tapered toward the forward end of the filter bag as shown in Fig. 10(a). The form of the filter bag used in Example 8 is illustratively shown in Fig. 32.

Using the produced multi-bag virus removal system, virus-removed plasma was prepared in substantially the same manner as in Example 1, except that the centrifugation for the filtration of the plasma component in the filter bag was performed under 500 G instead of 1000 G. With respect to the virus-removed plasma prepared (i.e., filtered plasma), it was found that the yield of the filtered plasma was 65 %.

The results above show that a filter bag which is tapered toward the forward end of the filter bag as viewed in a flow direction of the virus-containing suspension in the virus removal bag can achieve a yield of filtered plasma of larger than 60 %, even when the centrifugal force applied to the plasma is reduced to a half of the centrifugal force used in Example 1.

Example 9

Virus-removed plasma was prepared in substantially the same manner as in Example 1, except that the centrifugation for the filtration of the plasma component in the filter bag was performed under 500 G instead of 1000 G.

With respect to the virus-removed plasma prepared (i.e., filtered plasma), it was found that the yield of the filtered plasma was 45 %. The results show that, when the filter bag used is not tapered toward the forward end of the filter bag as viewed in a flow direction of the virus-containing suspension in the virus removal bag, a reduction of the centrifugal force applied to the plasma to a half of the centrifugal force used in Example 1 causes a drastic lowering of the yield of the filtered plasma.

Reference Example 3

A virus removal bag was produced in substantially the same manner as in Example 1, except that the size of the pouchy casing (a) made of flexible, soft polyvinyl chloride was changed to 13 cm $\times$ 13 cm. The volume of the second compartment (d) of the virus removal bag was 52 cm$^3$.

Using the thus produced virus removal bag, a multi -bag virus removal system was produced in the same manner as in Example 1. Then, using the multi-bag virus removal system, virus-removed plasma was prepared in substantially the same manner as in Example 1. With respect to the virus-removed plasma prepared (i.e., filtered plasma), it was found that the yield of the filtered plasma was only 41 %. Thus, it was found that a large portion of plasma component which was subjected to filtration remained unfiltered in the filter bag.

INDUSTRIAL APPLICABILITY

By the use of the virus removal bag of the present invention for removing viruses from a virus-containing suspension, a filtrate which is a virus-removed suspension is collected inside the virus removal bag and, therefore, there is no need to provide a separate container for receiving the filtrate. Accordingly, the virus removal bag of the present invention is advantageous not only in that it has a simple structure, but also in that the virus removal can be achieved by easy operations. Further, a virus removal system can be prepared easily by adding the virus removal bag of the present invention to a conventional multi-bag system used for blood treatment. The use of such a virus removal system for removing viruses from plasma is advantageous in that all viruses, including viruses in the window period and viruses which are not covered by the virus detection methods employed in the field of blood treatment, can be easily removed from plasma without any of performing complicated aseptic operations and using large scale apparatuses. Therefore, a transfusable plasma having very low risk of viral infection can be easily prepared at low cost.

**Claims**

1.  A virus removal bag for removing viruses from a virus-containing suspension, comprising:

    a pouchy casing (a) having at least one inlet for a virus-containing suspension and at least one outlet for a virus-removed suspension; and
    a separation membrane (b) which is securely held in said pouchy casing (a) and which partitions the internal space of said pouchy casing (a) into a first compartment (c) communicating with said inlet and a second compartment (d) communicating with said outlet,
    wherein at least a part of said separation membrane (b) is made of a virus removal membrane for removing viruses from a virus-containing suspension by filtration to obtain a filtrate which is a virus-removed suspension, and

    wherein said first compartment (c) serves to receive a virus-containing suspension introduced through said inlet, and said second compartment (d) serves to collect the filtrate obtained by filtering the virus-containing suspension through said virus removal membrane.

2.  The virus removal bag according to claim 1, wherein said separation membrane (b) is in the form of a membranous, overall surrounding wall of a filter bag,
    wherein said filter bag is securely held in said pouchy casing (a), such that the membranous, overall surrounding wall of said filter bag partitions the internal space of said pouchy casing (a) into the first compartment (c) which is the internal space of said filter bag communicating with said inlet and the second compartment (d) which is the internal space of said pouchy casing (a), exclusive of a filter bag portion, communicating with said outlet, and
    wherein at least a part of the surrounding wall of said filter bag is made of a virus removal membrane for removing viruses from the virus-containing suspension by filtration.

3.  The virus removal bag according to claim 2, wherein said filter bag is tapered toward the forward end of the filter bag as viewed in a flow direction of the virus-containing suspension in said virus removal bag, wherein the tapering begins at the backward end of said filter bag or at a portion during the course toward the forward end of said filter bag.

4.  The virus removal bag according to any one of claims 1 to 3, wherein said virus removal membrane is a composite filter in which at least one prefilter and at least one virus removal filter are laminated in this order as viewed in a flow direction of the virus-containing suspension, to thereby form a composite filter, wherein, on at least one side of the composite filter, a nonwoven fabric is provided.

5.  The virus removal bag according to any one of claims 1 to 4, wherein said virus removal membrane is a porous membrane having an average pore diameter in the range of from 1 to 100 nm.

6.  The virus removal bag according to any one of claims 1 to 5, wherein said virus removal membrane is a hydrophilic porous membrane obtained by an addition of a hydrophilic compound onto the surface of a porous membrane.

7.  The virus removal bag according to claim 6, wherein said addition of a hydrophilic compound is a graft polymerization reaction of a hydrophilic monomer.

8.  The virus removal bag according to any one of claims 1 to 7, which is flexible.

9.  The virus removal bag according to any one of claims 1 to 8, wherein said second compartment (d) has a volume sufficient to collect all of the filtrate obtained by filtering the virus-containing suspension through said virus removal membrane.

10. The virus removal bag according to any one of claims 1 to 9, wherein said first compartment (c) contains a spongy adsorber.

11. The virus removal bag according to any one of claims 1 to 10, wherein said second compartment (d) has a volume in the range of from 100 to 800 cm$^3$.

12. The virus removal bag according to any one of claims 1 to 11, which is aseptically and fluid-tightly connected to at least one functional bag which has a function other than a virus removal function, thereby providing a closed, multi-bag virus removal system.

13. A method for removing viruses from a virus -containing suspension, comprising:

(1) providing at least one virus removal bag of any one of claims 1 to 12;
(2) introducing a virus-containing suspension to said virus removal bag through said inlet, to receive said virus-containing suspension in said first compartment (c);
(3) filtering said virus-containing suspension through said virus removal membrane, to thereby remove viruses from said suspension;
(4) collecting a filtrate which is a virus -removed suspension in said second compartment (d); and
(5) withdrawing said virus-removed suspension through said outlet.

14. The method according to claim 13, wherein, in step (3), the filtration of said virus-containing suspension through said virus removal membrane is promoted by applying a centrifugal force to the virus-containing suspension in said first compartment (c).

15. The method according to claim 13, wherein, in step (3), the filtration of said virus-containing suspension through said virus removal membrane is promoted by applying a pressure to the virus-containing suspension in said first compartment (c).

16. The method according to any one of claims 13 to 15, wherein said virus-containing suspension is whole blood.

17. The method according to any one of claims 13 to 15, wherein said virus-containing suspension is plasma.

18. The method according to claim 17, wherein said plasma has never been frozen.

19. The method according to claim 17 or 18, wherein said plasma is leukocyte-removed plasma.

20. The method according to any one of claims 13 to 19, wherein, in step (4), all of the filtrate obtained by filtering the virus-containing suspension through said virus removal membrane is collected in said second compartment (d) before performing step (5).

21. A method for preparing a virus-removed plasma, comprising:

(1) providing a closed, virus removal system which comprises:

a plasma collector means (i) for collecting whole blood comprising plasma and blood cells and separating and collecting plasma from whole blood, wherein said whole blood is suspected to contain viruses,
at least one virus removal bag (ii) of any one of claims 1 to 11, and
a plasma recovery means (iii) for recovering a virus-removed plasma,
said plasma collector means (i) being aseptically and fluid-tightly connected to said virus removal bag (ii), and said virus removal bag (ii) being aseptically and fluid-tightly connected to said plasma recovery means (iii);

(2) collecting whole blood from a donor into said plasma collector means (i);
(3) separating the collected whole blood into plasma and blood cells by centrifugation;
(4) introducing the separated plasma into said at least one virus removal bag (ii) from said plasma collector means (i), to receive said separated plasma in said first compartment (c) of said virus removal bag (ii);
(5) filtering said separated plasma through said virus removal membrane of said virus removal bag (ii), to thereby remove viruses from said separated plasma;
(6) collecting a filtrate which is a virus -removed plasma in said second compartment (d) of said virus removal bag (ii); and
(7) discharging the virus-removed plasma from said virus removal bag (ii) into said plasma recovery means (iii).

22. The method according to claim 21, wherein, in step (6), all of the filtrate obtained by filtering said separated plasma is collected in said second compartment (d) of said virus removal bag (ii) before performing step (7).

**23.** Human or animal plasma obtained by the method of claim 21 or 22.

# Fig. 1

# Fig. 2(a)

# Fig. 2(b)

# Fig. 3

# Fig. 4

# Fig. 5

## Fig. 6(a)

## Fig. 6(b)

## Fig. 6(c)

## Fig. 6(d)

# Fig. 7(a)

# Fig. 7(b)

# Fig. 7(c)

Fig. 8(a)

Fig. 8(b)

Fig. 9(a)

Fig. 9(b)

Fig. 10(a)

Fig. 10(b)

Fig. 10(c)

Fig. 10(d)

Fig. 10(e)

Fig. 10(f)

## Fig. 11(a)

## Fig. 11(b)

## Fig. 12(a)

## Fig. 12(b)

## Fig. 13

## Fig. 14

# Fig. 15

# Fig. 16

# Fig. 17

18

24

20

1

21

25

# Fig. 18

18

22

20

23

1

21

24

25

# Fig. 19

Fig. 20

## Fig. 21

## Fig. 22

# Fig. 23

# Fig. 24(a)

# Fig. 24(b)

# Fig. 25(a)

# Fig. 25(b)

EP 1 579 838 A1

## Fig. 25(c)

## Fig. 25(d)

# Fig. 26

## Fig. 27

## Fig. 28

# Fig. 29

# Fig. 30

# Fig. 31

# Fig. 32

## INTERNATIONAL SEARCH REPORT

| | International application No. |
| --- | --- |
| | PCT/JP03/15974 |

**A. CLASSIFICATION OF SUBJECT MATTER**
Int.Cl$^7$ A61J1/05

According to International Patent Classification (IPC) or to both national classification and IPC

**B. FIELDS SEARCHED**

Minimum documentation searched (classification system followed by classification symbols)
Int.Cl$^7$ A61J1/05-1/22, B01D39/00-39/20

Documentation searched other than minimum documentation to the extent that such documents are included in the fields searched

| | | | |
| --- | --- | --- | --- |
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 11-267421 A (Terumo Corp.), 05 October, 1999 (05.10.99), Full text; all drawings (Family: none) | 1-23 |
| A | WO 01/28597 A1 (MACO PHARMA.), 26 April, 2001 (26.04.01), Full text; all drawings & EP 1093823 A1 & JP 2003-512093 A | 1-23 |
| A | JP 3-146067 A (Asahi Chemical Industry Co., Ltd.), 21 June, 1991 (21.06.91), Full text; all drawings (Family: none) | 1-23 |

☒ Further documents are listed in the continuation of Box C. ☐ See patent family annex.

| | |
| --- | --- |
| * Special categories of cited documents: <br> "A" document defining the general state of the art which is not considered to be of particular relevance <br> "E" earlier document but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| 12 March, 2004 (12.03.04) | 30 March, 2004 (30.03.04) |

| Name and mailing address of the ISA/ <br> Japanese Patent Office | Authorized officer |
| --- | --- |
| Facsimile No. | Telephone No. |

Form PCT/ISA/210 (second sheet) (July 1998)

**EP 1 579 838 A1**

**INTERNATIONAL SEARCH REPORT**

International application No.

PCT/JP03/15974

C (Continuation). DOCUMENTS CONSIDERED TO BE RELEVANT

| Category* | Citation of document, with indication, where appropriate, of the relevant passages | Relevant to claim No. |
|---|---|---|
| A | US 5100564 A  (Pall Corp.),<br>31 March, 1992 (31.03.92),<br>Full text; all drawings<br>& WO 92/07656 A          & EP 556303 A<br>& JP 2570906 B2 | 1-23 |

Form PCT/ISA/210 (continuation of second sheet) (July 1998)

52

# INTERNATIONAL SEARCH REPORT

| | International application No. |
|---|---|
| | PCT/JP03/15974 |

**Box I   Observations where certain claims were found unsearchable (Continuation of item 2 of first sheet)**

This international search report has not been established in respect of certain claims under Article 17(2)(a) for the following reasons:

1. ☒ Claims Nos.: 13 to 22
   because they relate to subject matter not required to be searched by this Authority, namely:
   The inventions as set forth in claims 13 to 22 are relevant to methods for treatment of the human body by surgery or therapy and diagnostic methods.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II   Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐   The additional search fees were accompanied by the applicant's protest.

☐   No protest accompanied the payment of additional search fees.

Form PCT/ISA/210 (continuation of first sheet (1)) (July 1998)